# EUROPÄISCHE PATENTSCHRIFT

(11) **EP 0 584 091 B1**
(45) Veröffentlichungstag und Bekanntmachung des Hinweises auf die Patenterteilung: **15.01.1997**
(21) Anmeldenummer: 92908141.2
(22) Anmeldetag: 02.04.1992
(51) Int. Cl.: C07D 311/16, C07D 405/12, C07D 311/94, C07D 311/50, C07D 311/80, C07D 407/12

(54) **BENZOPYRANONE, VERFAHREN ZU IHRER HERSTELLUNG UND VERWENDUNG**
BENZOPYRANONES, METHODS OF MANUFACTURE AND USE THEREOF
BENZOPYRANONES, PROCEDES DE FABRICATION ET UTILISATION

(30) Priorität: 11.04.1991 DE 4111861
(43) Veröffentlichungstag der Anmeldung: 02.03.1994
(73) Patentinhaber: Dr. Willmar Schwabe GmbH & Co., D-76209 Karlsruhe (DE)
(72) Erfinder: CHATTERJEE, Shyam, Sunder, D-7500 Karlsruhe 1 (DE); NÖLDNER, Michael, 76229 Eggenstein (DE); HAUER, Hermann, D-7500 Karlsruhe (DE); KOCH, Egon, 76229 Karlsruhe (DE)
(74) Vertreter: Bunke, Holger, Dr.rer.nat. Dipl.-Chem.
(86) Internationale Anmeldenummer: EP9200739
(87) Internationale Veröffentlichungsnummer: WO9218493

(56) Entgegenhaltungen:
- EP-A- 0 043 535
- EP-A- 0 171 645
- EP-A- 0 175 541
- DE-A- 2 123 924
- US-A- 4 569 994
- JOURNAL OF MEDICINAL CHEMISTRY, Band 11, Nr. 2, 26 Februar 1968, Washington, DC (US); J.I. DEGRAW et al., Seiten 375-376
- CHEMICAL ABSTRACTS, Band 72, Nr. 7, 16 Februar 1970, Columbus, OH (US); V.A. ZAGOREVSKI et al., Seite 303, Nr. 31539y
- CHEMICAL & PHARMACEUTICAL BULLETIN, Band 38, Nr. 3, März 1990, Tokyo (JP); K. AIHARA et al., Seiten 842-844
- JOURNAL OF BIOLOGICAL CHEMISTRY, Band 259, Nr. 5, 10. März 1984, Baltimore, MA (US); N. HARADA et al., Seiten 3005-3010
- JOURNAL OF THE CHEMICAL SOCIETY, CHEMICAL COMMUNICATIONS, Nr. 7, April 1983, Letchworth (GB); S.V KESSAR et al., 400-401

## Beschreibung

Die Erfindung betrifft Benzopyranone, deren Grundstruktur sich von Cumarin ableiten läßt, Verfahren zur Herstellung dieser Verbindungen, einschließlich der dabei entstehenden reaktiven Zwischenprodukte, sowie Arzneimittel, die diese Verbindungen enthalten.

Das Zentralnervensystem (ZNS) von Säugetieren besitzt hohe Konzentrationen von exzitatorischen Aminosäuren (EAS) wie Glutamat, Aspartat und Homocysteat, die als Neurotransmitter fungieren, welche mit spezifischen Rezeptoren zusammenwirken.

Die drei am besten charakterisierten Rezeptor-Typen sind die nach ihren selektiven Agonisten benannten N-Methyl-D-aspartat-(NMDA), Kainat- (KA) und Quisqualat- (QA) Rezeptoren. Alle drei Rezeptoren können durch Glutamat und Aspartat aktiviert werden. Es ist bekannt, daß als Folge cerebraler Ischämien Glutamat in größerer Menge freigesetzt wird, welches u.a. am NMDA-Rezeptorkomplex bindet und zu einem vermehrten Calciumeinstrom sowie zu einer erhöhten Freisetzung intrazellulären Calciums in den neuronalen Zellen führt. Der NMDA-Rezeptorkomplex beinhaltet u.a. Bindungsstellen für Glutamat, Glycin, Phencyclidin, Mg²⁺ und Zn²⁺. Da eine Reihe von pharmakologischen Befunden darauf hindeuten, daß Modulatoren der NMDA-Rezeptor-vermittelten Neurotransmission die NMDA-vermittelte Zytotoxizitat beeinflussen können, wurden bereits verschiedene selektive NMDA-Antagonisten im Hinblick auf ihre mögliche neuroprotektive Wirkung untersucht (vgl. G.L. Collingridge, R.A.J. Lester: "Excitatory Amino Acid Receptors in the Vertebrate Central Nervous System", Pharmacol. Rev. 40, No. 2, pp. 143-210 (1989); L. Turski: "N-Methyl-D-aspartat-Rezeptorkomplex", Arzneim.-Forsch./Drug Res. 40 (I), Nr. 5, S. 511-514 (1990)). Wegen unerwünschter Nebenwirkungen der bekannten NMDA-Antagonisten besteht weiterhin ein starkes Bedürfnis nach der Bereitstellung neuer Verbindungen mit NMDA-antagonistischer Wirkung, die geringere Nebenwirkungen oder ein anderes Wirkungsspektrum zeigen.

Aus Journal of Medicinal Chemistry (USA), Volume 11, S. 375 bis 376, (1968), sind in 3- und/oder 4-Stellung alkylierte Derivate von 6,7-Dihydroxy-2H-1-benzopyran-2-on und 6-Methoxy-7-hydroxy-2H-1-benzopyran-2-on bekannt, die aus einem entsprechenden β-Ketoester durch Kondensation mit 3-Hydroxy-4-methoxyphenylformiat in Orthophosphorsäure und ggf. nachfolgender Etherspaltung hergestellt werden. Diese bekannten Benzopyranone wirken teilweise als Inhibitoren für das Enzym Phenylalaninhydroxylase und können somit verwendet werden, um experimentell Phenylketonurie bei Ratten zu induzieren.

Der Erfindung liegt die Aufgabe zugrunde, neue Verbindungen zu schaffen, die eine möglichst geringe Toxizität, aber dennoch NMDA-antagonistische Wirkung besitzen und als Arzneimittelwirkstoffe, insbesondere in der Therapie chronischer neurodegenerativer Erkrankungen, verwendet werden können, um durch Ischämie/Trauma oder durch andere pathologische Veränderungen verursachte Neurodegenerationen im ZNS und das Auftreten von Konvulsionen zu verhindern oder doch mindestens zu verringern. Außerdem können die neuen Verbindungen zusätzlich ein breites antiallergisches und entzündungshemmendes Profil mit langer Wirkungsdauer zeigen; sie sollen dabei aber oral applizierbar sein.

Diese Aufgabe wird durch die Bereitstellung der erfindungsgemäßen Verbindungen und Verfahren sowie durch die Verwendung dieser Verbindungen als neuroprotektiv, antikonvulsiv, antiepileptisch und ggf. antiallergisch und antiinflammatorisch wirksame Arzneimittel gelöst.

Gegenstand der Erfindung sind somit:

2H-1-Benzopyran-2-one der allgemeinen Formel I, 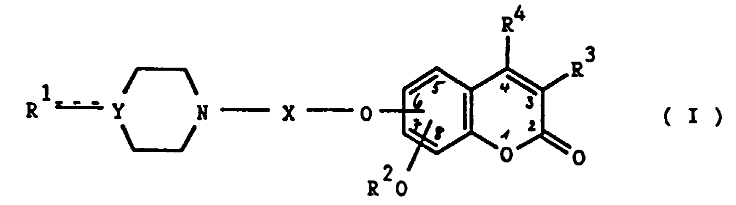 worin
- X: eine Alkylengruppe mit 2 bis 5 C-Atomen oder eine 2-Hydroxypropylengruppe,
- Y: ein Stickstoffatom, eine CH-Gruppe, eine COH-Gruppe oder ein Kohlenstoffatom,
- R¹: einen Phenyl-, Benzhydryl-, Benzhydryliden-, Benzyl-, Diphenylhydroxymethyl-, Pyridinyl- oder Pyrimidinyl-Rest, der gegebenenfalls mit jeweils ein oder zwei C₁-C₅-Alkylgruppen, mit jeweils ein oder zwei Halogenatomen, mit Halogen und gleichzeitig C₁-C₅-Alkyl, mit Perfluoralkyl mit 1 bis 3 C-Atomen, C₁-C₅-Alkoxy, Methylendioxy, Hydroxy oder Nitro substituiert ist,
- R²: einen geradkettigen oder verzweigten Alkylrest mit 1 bis 5 C-Atomen oder einen Cycloalkylrest mit 4 bis 6 C-Atomen,
- R³: ein Wasserstoffatom, eine Alkylgruppe mit 1 bis 4 C-Atomen oder Phenyl,
- R⁴: ein Wasserstoffatom, eine Alkylgruppe mit 1 bis 4 C-Atomen, einen Phenylrest oder einen Trifluormethylrest oder
- R³ und R⁴: gemeinsam eine Polymethylenkette -(CH₂)ₙ- mit n = 3 oder 4
darstellen, sowie deren Additionsverbindungen mit physiologisch verträglichen Säuren.

Die erfindungsgemäßen Verbindungen sind neu. Bereits bekannt sind Verbindungen, die vom 7-Hydroxycumarin (Umbelliferon) abgeleitet sind, denen jedoch eine zweite Alkoxygruppe, entsprechend dem Rest R²O- der allgemeinen Formel I, fehlt. Solche Umbelliferonderivate sind in DE-C3-2123924 und EP-A1-0171645 beschrieben, und zwar auch als antiödematöse (nur DE 2123924), entzündungswidrige und antiallergische Wirkstoffe von Arzneimitteln, insbesondere Antihistaminika. Ferner werden in EP-A1-0175541 ähnliche Umbelliferonderivate mit antipsychotischer und anxiolytischer Wirksamkeit, sowie in US-A-4569994 solche mit antihypoxischer Wirksamkeit beschrieben.

Schließlich sind in der EP-B1-0043535 tricyclische Verbindungen der allgemeinen Formel I beschrieben, bei denen jedoch R¹ immer ein gegebenenfalls substituierter Aminorest ist und die sich damit von den erfindungsgemäßen Verbindungen erheblich unterscheiden. Diese bekannten Verbindungen werden als antiallergisch, antiödematös und antiphlogistisch wirksam bezeichnet und sollen vorzugsweise bei der Bekämpfung von Asthma, Heuschnupfen und Urticaria eingesetzt werden.

Im Hinblick auf diesen Stand der Technik war es für den Fachmann überraschend, daß die erfindungsgemäßen Verbindungen der allgemeinen Formel I eine NMDA-antagonistische und neuroprotektive Wirkung aufweisen, wobei einige von ihnen zusätzlich auch antiallergisch und antiinflammatorisch wirksam sind.

Bei den erfindungsgemäßen Verbindungen der allgemeinen Formel I können die beiden, an den anellierten Benzolring des Cumaringerüstes gebundenen Alkoxyreste, also der Rest R²O- und der Rest mit der allgemeinen Formel II 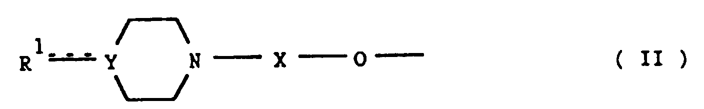 worin R¹, Y und X die oben genannten Bedeutungen besitzen, in beliebigen Kombinationen an den Positionen 5 bis 8 des Cumaringerüstes gebunden sein. Bevorzugt sind dabei solche Verbindungen der allgemeinen Formel I, bei denen der Rest II in 6- oder 7-Position und der Alkoxyrest R²O- in der dementsprechend noch freien 7- oder 6-Position des 2H-1-Benzopyran-2-ons gebunden sind.

Erfindungsgemäß bevorzugte Verbindungen der allgemeinen Formel I sind solche, bei denen
- X: eine Alkylengruppe mit 2 bis 5 C-Atomen,
- Y: N oder CH,
- R¹: einen ggf. mit einem Halogenatom, mit Methyl, Methoxy, Ethoxy oder Hydroxy substituierten Phenyl- oder Diphenylhydroxymethylrest,
- R²: Methyl oder Ethyl,
- R³: ein Wasserstoffatom oder eine Methyl- oder Phenylgruppe,
- R⁴: ein Wasserstoffatom, Methyl, Propyl, Isopropyl oder
- R³ und R⁴: gemeinsam eine Polymethylenkette -(CH₂)ₙ- mit n = 3 oder 4
bedeuten, sowie deren Additionsverbindungen mit physiologisch verträglichen Säuren.

Wegen ihrer pharmakologischen Wirksamkeit besonders bevorzugte Verbindungen sind diejenigen der Beispiele 1-4, 7, 14, 17, 29, 33, 35, 41-49, 52, 54-60 und 66, d.h. 7-Methoxy-6-[3-(4-phenyl-1-piperazinyl)propoxy]-2H-1-benzopyran-2-on, 6-Methoxy-7-[3-(4-phenyl-1-piperazinyl)propoxy]-2H-1-benzopyran-2-on, 7-Ethoxy-6-[3-(4-phenyl-1-piperazinyl)propoxy]-2H-1-benzopyran-2-on, 6-Ethoxy-7-[3-(4-phenyl-1-piperazinyl)propoxy]-2H-1-benzopyran-2-on, 7-Ethoxy-6-{3-[4-(2-methoxyphenyl)-1-piperazinyl]-propoxy}-2H-1-benzopyran-2-on, 7-{3-[4-(Bis(4-fluorphenyl)-hydroxymethyl)-1-piperidinyl]propoxy}-6-methoxy-2H-1-benzopyran-2-on, 7-{3-[4-(Diphenylhydroxymethyl)-1-piperidinyl]-propoxy}-6-methoxy-2H-1-benzopyran-2-on, 7-Methoxy-3,4-dimethyl-6-[3-(4-phenyl-1-piperidinyl)propoxy]-2H-1-benzopyran-2-on, 7-Methoxy-6-[3-(4-phenyl-1-piperazinyl)propoxy]-4-propyl-2H-1-benzopyran-2-on, 7-Methoxy-4-(1-methylethyl)-6-[3-(4-phenyl-1-piperazinyl)propoxy]-2H-1-benzopyran-2-on, 7-Methoxy-4-methyl-3-phenyl-6-[3-(4-phenyl-1-piperazinyl)propoxy]-2H-1-benzopyran-2-on, 7,8,9,10-Tetrahydro-3-methoxy-2-[3-(4-phenyl-1-piperazinyl)propoxy]-6H-dibenzo[b,d]pyran-6-on, 7,8,9,10-Tetrahydro-3-methoxy-2-[3-(4-phenyl-1-piperidinyl)propoxy]-6H-dibenzo[b,d]pyran-6-on, 2,3-Dihydro-7-methoxy-8-[2-(4-phenyl-1-piperazinyl)ethoxy]cyclopenta[c][1]benzopyran-4(1H)-on, 2,3-Dihydro-7-methoxy-8-[3-(4-phenyl-1-piperazinyl)propoxy]cyclopenta[c][1]benzopyran-4(1H)-on, 2,3-Dihydro-7-methoxy-8-[4-(4-phenyl-1-piperazinyl)butyloxy]cyclopenta[c][1]benzopyran-4(1H)-on, 2,3-Dihydro-7-methoxy-8-[5-(4-phenyl-1-piperazinyl)pentyloxy]cyclopenta[c][1]benzopyran-4(1H)-on, 2,3-Dihydro-7-methoxy-8-[2-(4-phenyl-1-piperidinyl)ethoxy]cyclopenta[c][1]benzopyran-4(1H)-on, 2,3-Dihydro-7-methoxy-8-[3-(4-phenyl-1-piperidinyl)-propoxy]cyclopenta[c][1]benzopyran-4(1H)-on, 8-{3-[4-(4-Fluorphenyl)-1-piperazinyl]propoxy}-2,3-dihydro-7-methoxy-cyclopenta[c][1]benzopyran-4(1H)-on, 2,3-Dihydro-7-methoxy-8-{3-[4-(2-methoxyphenyl)-1-piperazinyl]propoxy}cyclopenta[c][1]benzopyran-4(1H)-on, 2,3-Dihydro-7-methoxy-8-{3-[4-(3-methoxyphenyl)-1-piperazinyl]propoxy}cyclopenta[c][1]benzopyran-4-(1H)-on, 2,3-Dihydro-7-methoxy-8-{3-[4-(4-methoxyphenyl)-1-piperazinyl]propoxy)cyclopenta[c][1]benzopyran-4(1H)-on, 8-{3-[4-(2-Ethoxyphenyl)-1-piperazinyl]propoxy}-2,3-dihydro-7-methoxy-cyclopenta[c][1]benzopyran-4(1H)-on, 8-{3-[4-(2-Chlorphenyl)-1-piperazinyl]propoxy}-2,3-dihydro-7-methoxy-cyclopenta[c] [1]benzopyran-4(1H)-on, 2,3-Dihydro-7-methoxy-8-{3-[4-(2-methylphenyl)-1-piperazinyl]propoxy}cyclopenta[c][1]benzopyran-4(1H)-on, 2,3-Dihydro-8-{3-[4-(2-hydroxyphenyl)-1-piperazinyl]propoxy}-7-methoxy-cyclopenta[c][1]benzopyran-4(1H)-on, 8-{3-[4-(2-Fluorphenyl)-1-piperazinyl]propoxy}-2,3-dihydro-7-methoxy-cyclopenta[c][1]benzopyran-4(1H)-on.

Bei dem erfindungsgemäßen Verfahren zur Herstellung der Verbindungen der allgemeinen Formel I wird entweder
a) eine Verbindung der allgemeinen Formel III 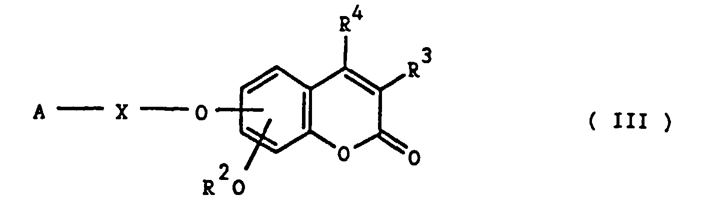 worin X, R², R³ und R⁴ die oben angegebenen Bedeutungen haben und A eine Abgangsgruppe ist, die ausgewählt ist aus der Gruppe Chlor, Brom, Iod, Alkylsulfonyloxy, Trifluormethylsulfonyloxy, gegebenenfalls mit Alkyl, Nitro oder Halogen substituiertes Phenylsulfonyloxy,
   mit einer Verbindung der allgemeinen Formel V umgesetzt, 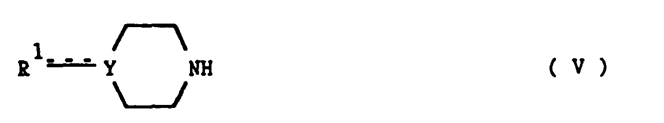 in der Y und R¹ die oben angegebenen Bedeutungen haben, wobei die Verbindung der allgemeinen Formel V auch in Form ihres Hydrochlorids oder eines anderen Säureadditionssalzes vorliegen kann,
   oder
b) eine Verbindung der allgemeinen Formel VI, 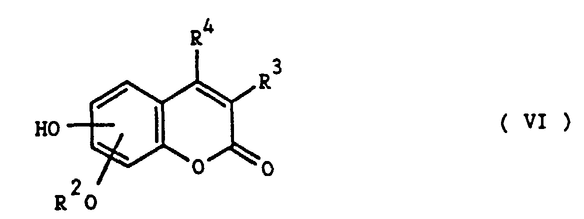 worin R², R³ und R⁴ die oben angegebenen Bedeutungen haben, wird mit einer Verbindung der allgemeinen Formel VII umgesetzt, 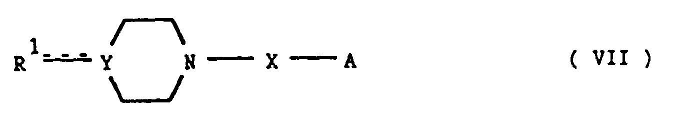 in der A, X, Y und R¹ die oben angegebenen Bedeutungen haben,
   worauf sowohl bei (a) als auch bei (b) die erhaltenen Produkte ggf. in ihre physiologisch verträglichen Säureadditionsverbindungen übergeführt werden.

Die Umsetzungen nach a) oder b) werden in an sich bekannter Weise durchgeführt. So wird z.B. in der DE-PS 2123924 die Reaktion von 7-Hydroxy-3,4-dimethylcumarin mit 1-(3-Chlorpropyl)-4-benzyl-piperazin beschrieben. Zur Bindung der entstehenden Säure HA, worin A eine der vorstehend definierten Abgangsgruppen ist, finden diese Reaktionen in Gegenwart einer Base wie z.B. eines Alkali- oder Erdalkalicarbonates, -hydrogencarbonates, -hydrids, -alkoholats, -hydroxyds oder eines tertiären Amins statt, wobei vorzugsweise Alkalicarbonate und -hydrogencarbonate oder Alkalihydride zum Einsatz kommen. Die Umsetzungen werden vorteilhaft in Gegenwart von gegenüber den Reaktanden inerten Lösungsmitteln durchgeführt. Gut geeignet sind hierfür Alkanole, aromatische Lösungsmittel wie Toluol, Xylol usw., oder dipolare aprotische Lösungsmittel wie aliphatische oder cycloaliphatische Ether, Carbonsäuredialkylamide, Tetraalkylharnstoffe, Ketone und Sulfoxide. Bevorzugt sind Alkanole mit 1 bis 5 C-Atomen und Dimethylformamid. Gegebenenfalls werden 0.02 bis 0.5 Äquivalente eines Alkali- oder Erdalkaliiodids, vorzugsweise 0.05 bis 0.2 Äquivalente Kaliumiodid, als Katalysator zugesetzt. Die Umsetzung kann bei einer Temperatur durchgeführt werden, die zwischen Raumtemperatur und 130°C, bevorzugt jedoch zwischen Raumtemperatur und 100°C bzw., bei niedersiedenden Lösungsmitteln, in der Nähe des Siedepunkts des Lösungsmittels liegt. Oxidative Nebenprodukte lassen sich durch Arbeiten unter einer Schutzgasatmosphäre, beispielsweise Stickstoff oder Argon, vermeiden. Die Reaktionen werden unter Normaldruck oder in geschlossenen Gefäßen bei Drücken bis zu etwa 10⁷ Pa (100 bar) durchgeführt.

Diejenigen Verbindungen der allgemeinen Formel I, bei denen X eine 2-Hydroxypropylengruppe ist, können gemäß einer weiteren Variante des erfindungsgemäßen Verfahrens dadurch hergestellt werden, daß man eine Epoxid-Verbindung der allgemeinen Formel IV, 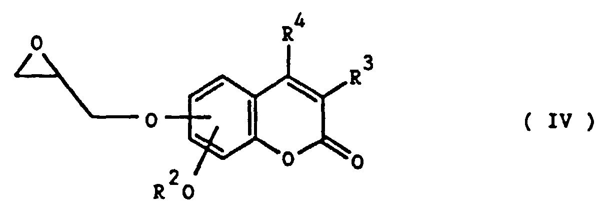 worin R², R³ und R⁴ die oben genannten Bedeutungen besitzen, mit einer Verbindung der allgemeinen Formel V umsetzt, 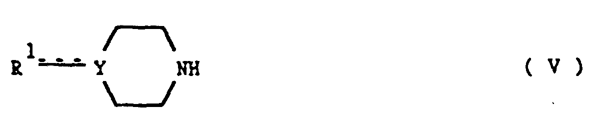 worin R¹ und Y die oben genannten Bedeutungen besitzen, wobei die Verbindung der allgemeinen Formel V auch in Form ihres Hydrochlorids oder eines anderen Säureadditionssalzes vorliegen kann.

Im Gegensatz zu den Varianten (a) und (b) wird bei dieser Variante keine Abgangsgruppe A benötigt, weshalb keine Säure entsteht und sich ein Basenzusatz erübrigt. Lösungsmittel, Katalysatoren, Temperaturen, Drücke und sonstige Verfahrensbedingungen sind dagegen bei allen Varianten gleich.

Die Herstellung der erfindungsgemäßen Verbindungen der allgemeinen Formel I kann über reaktive Zwischenprodukte der allgemeinen Formel XI erfolgen, 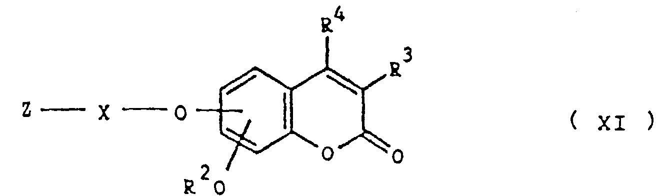 worin R², R³ und R⁴ die in Anspruch 1 angegebenen Bedeutungen haben, und
- X: eine Alkylengruppe mit 2 bis 5 C-Atomen und
- Z: eine Hydroxyl- oder Methansulfonyloxygruppe, ein Halogenatom oder
- Z-X: gemeinsam eine Oxiranylmethylengruppe
bedeuten.

Auf diese Zwischenprodukte, ihre Herstellung und Weiterverarbeitung wird nachfolgend näher eingegangen. Die Reste Z-X- und R²O- können wiederum in beliebigen Kombinationen an den Positionen 5 bis 8 des Cumaringerüstes gebunden sein, wobei die Positionen 6 und 7 bevorzugt sind.

Die Verbindungen der allgemeinen Formel III können dadurch hergestellt werden, daß man eine Verbindung der allgemeinen Formel VI, in der R², R³ und R⁴ die oben angegebenen Bedeutungen haben, entweder mit Alkyldihalogeniden Hal-X-Hal umsetzt, worin X eine Alkylengruppe mit 2-5 C-Atomen bedeutet, oder mit Halogenalkoholen Hal-X-OH, worin X eine Alkylengruppe mit 2 bis 5 C-Atomen bedeutet, erst zu Verbindungen der allgemeinen Formel VIII umsetzt, 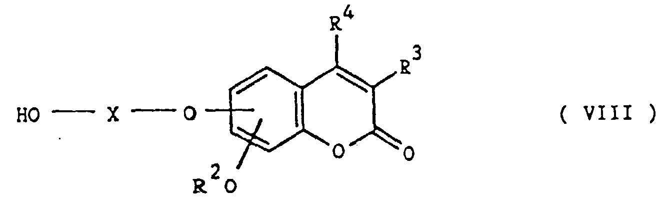 worin X, R², R³ und R⁴ die im Zusammenhang mit der allgemeinen Formel I angegebenen Bedeutungen besitzen, und anschließend mit einem Sulfonsäurechlorid in an sich bekannter Weise umsetzt. Die Umsetzungen mit den genannten Halogenverbindungen entsprechen den oben beschriebenen Verfahrensvarianten (a) und (b). Man verwendet die dort beschriebenen Basen, Lösungsmittel, Katalysator, Reaktionstemperaturen, Schutzgasatmosphäre und Druckbedingungen. Bevorzugt wird hier jedoch ein Alkalihydrid, -carbonat oder -hydrogencarbonat als Base und Dimethylformamid oder Butanon(-2) als Lösungsmittel. Die Umsetzungstemperatur liegt bevorzugt zwischen 60°C und 100°C.

Bei der Umsetzung der Verbindung der allgemeinen Formel VIII mit einem Sulfonsäurechlorid werden in an sich bekannter Weise die Sulfonylverbindungen der Formel III mit den oben angegebenen Bedeutungen für X und R², R³ und R⁴ und mit Alkylsulfonyloxy, Trifluormethylsulfonyloxy oder gegebenenfalls mit Alkyl, Nitro oder Halogen substituiertem Phenylsulfonyloxy als Abgangsgruppe A gebildet. Dazu setzt man den entsprechenden Alkohol der Formel VIII mit einem Alkyl-, Trifluormethyl- oder gegebenenfalls mit einem durch Alkyl, Nitro oder Halogen substituierten Phenyl-sulfonsäurehalogenid, vorzugsweise mit Methan- oder p-Toluolsulfonsäurechlorid, um. Zur Bindung der dabei entstehenden Halogenwasserstoff-Säure finden diese Reaktionen in Gegenwart einer Base wie z.B. eines Alkali- oder Erdalkalicarbonates oder -hydrogencarbonates oder eines tertiären oder aromatischen Amins statt, wobei vorzugsweise tertiäre Amine wie Triethylamin zum Einsatz kommen. Die Umsetzungen werden vorteilhaft in Gegenwart von gegenüber den Reaktanden inerten Lösungsmitteln durchgeführt. Gut geeignet sind hierfür aromatische Lösungsmittel wie Toluol, Xylol usw., oder dipolare aprotische Lösungsmittel wie aliphatische oder cycloaliphatische Ether, Carbonsäuredialkylamide, Tetraalkylharnstoffe, Ketone, Sulfoxide und halogenierte Alkane. Bevorzugt sind halogenierte Alkane wie Chloroform oder Dichlormethan. Die Reaktionstemperatur kann zwischen -30°C und +60°C, bevorzugt jedoch zwischen 0°C und +30°C, liegen. Oxidative Nebenprodukte lassen sich durch Arbeiten unter Schutzgasatmosphäre, beispielsweise Stickstoff oder Argon, vermeiden.

Die Verbindungen der allgemeinen Formel IV können in entsprechender Weise wie diejenigen der Formel III dadurch hergestellt werden, daß man eine Verbindung der allgemeinen Formel VI, in der R^{2,} R³ und R⁴ die oben angegebenen Bedeutungen haben, mit Epichlorhydrin oder Epibromhydrin umsetzt, wobei die gleichen Reaktionsbedingungen eingehalten werden, wie bei den Varianten (a) und (b) angegeben.

Die Verbindungen der allgemeinen Formel IX, 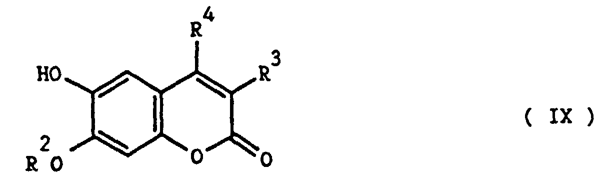 worin R², R³ und R⁴ die oben angegebenen Bedeutungen haben, R⁴ jedoch nicht Wasserstoff bedeutet, können dadurch hergestellt werden, daß man ein 2-Alkoxyhydrochinon R²O-C₆H₃(OH)₂ mit einem β-Ketocarbonsäureester der allgemeinen Formel X, 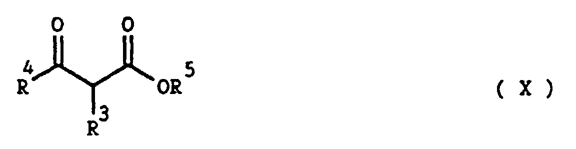 in der R³ und R⁴ die vorstehend genannten Bedeutungen haben und R⁵ ein Niederalkylrest ist, in an sich bekannter Weise in Gegenwart eines sauren Katalysators umsetzt. Als sauren Katalysator verwendet man Mineral- oder Lewissäuren ohne Lösungsmittel oder in gegenüber der Reaktion inerten Lösungsmitteln wie z.B. Alkoholen oder Eisessig, vorzugsweise 50- bis 100%ige Schwefelsäure ohne weiteres Lösungsmittel. Die Reaktion wird bei 0°C bis 60°C, vorzugsweise bei 0 bis 25°C durchgeführt.

überraschenderweise verläuft diese Reaktion regioselektiv. Die ebenso als mögliche Reaktionsprodukte denkbaren 5-Alkoxy-6-hydroxy- und 8-Alkoxy-6-hydroxy-cumarin-Derivate wurden in keinem Fall beobachtet.

Die für die Synthese der Verbindungen der allgemeinen Formel I verwendeten Zwischenprodukte der allgemeinen Formeln III, IV, VI, VIII und IX mit den oben angegebenen Bedeutungen für A, X, R², R³ und R⁴ sind mit wenigen Ausnahmen neu. Die neuen Zwischenprodukte, insbesondere diejenigen der allgemeinen Formel IX, sind deshalb ebenfalls Gegenstand der Erfindung. Bekannt sind dagegen die durch Disclaimer vom Schutz ausgenommenen Verbindungen der allgemeinen Formel XI, nämlich 7-Methoxy-6-(oxiranylmethoxy)-2H-1-benzopyran-2-on und 7-Methoxy-4-methyl-6-(oxiranylmethoxy)-2H-1-benzopyran-2-on.

Die erfindungsgemäßen Verbindungen können, sofern sie ein oder mehrere Asymmetriezentren enthalten, in Form von Racematen, Enantiomeren oder Diastereomeren vorliegen. Jede dieser Formen ist für sich allein oder im Gemisch mit einer oder mehreren der anderen Formen Gegenstand der Erfindung.

Gegenstand der Erfindung sind ferner Arzneimittel, die als Wirkstoff eine oder mehrere der erfindungsgemäßen Verbindungen der allgemeinen Formel I sowie gegebenenfalls zusätzlich pharmakologisch inerte Hilfsstoffe wie zum Beispiel Wasser, pflanzliche Öle, Polyethylenglykole, Glycerinester, Gelatine, Kohlenhydrate wie Lactose oder Stärke, Magnesiumstearat, Talk, Vaseline®, Konservierungsmittel, Netzmittel, Emulgatoren, physiologisch unbedenkliche Salze, Puffersubstanzen, Farbstoffe, Geschmacksstoffe und Aromastoffe enthalten. Die Wahl der Begleitstoffe hängt von der gewünschten Applikationsform wie z.B. Tabletten, Dragees, Säfte, Ampullen, Suppositorien, Salben oder Sprays ab. Die erfindungsgemäßen Verbindungen können auch im Gemisch mit anderen bekannten Wirkstoffen verabreicht werden.

In den nachfolgenden Beispielen sind die erfindungsgemäßen Verbindungen, die Verfahren zu ihrer Herstellung sowie die pharmakologischen Versuchsergebnisse näher beschrieben.

### I. Beispiele 1 bis 66 für Endprodukte der allgemeinen Formel I

Zur Herstellung der in den nachfolgenden Beispielen 1 bis 66 näher beschriebenen Verbindungen wurden folgende Verfahren benutzt:

Verfahren A: 5 bis 10 g des entsprechend mit R²O, R³ und R⁴ substituierten Methansulfonyloxyalkoxy-2H-1-benzopyran-2-ons oder Halogenalkoxy-2H-1-benzopyran-2-ons werden mit 1.0 bis 1.5 Äquivalenten des gewünschten mit R¹ substituierten Piperidins oder Piperazins in Form der Base oder des Hydrochlorids, mit 2.0 Äquivalenten Kaliumcarbonat und mit etwa 1 g Kaliumiodid in etwa 200 ml Dimethylformamid 4 bis 60 h unter Stickstoff bei 40 bis 80°C gerührt. Man entfernt das Lösungsmittel im Vakuum, nimmt in Chloroform oder Ethylacetat auf, wäscht gegebenenfalls mit verdünnter Natronlauge, dann mit Wasser, trocknet über Natriumsulfat und entfernt erneut das Lösungsmittel. Man reinigt durch Umkristallisation oder säulenchromatographisch über Kieselgel.

Verfahren B: 4 bis 11 g des entsprechend mit R²O, R³ und R⁴ substituierten Methansulfonyloxyalkoxy-2H-1-benzopyran-2-ons oder Halogenalkoxy-2H-1-benzopyran-2-ons werden mit einem Äquivalent des gewünschten mit R¹ substituierten Piperidins oder Piperazins in Form der Base oder des Hydrochlorids, mit 2.0 Äquivalenten Kaliumhydrogencarbonat und mit etwa 1 g Kaliumiodid in etwa 200 ml Dimethylformamid 4 bis 75 h unter Stickstoff bei 25 bis 90 °C gerührt. Man entfernt das Lösungsmittel im Vakuum, nimmt in Chloroform oder Ethylacetat auf, wäscht gegebenenfalls mit verdünnter Natronlauge, dann mit Wasser, trocknet über Natriumsulfat und entfernt erneut das Lösungsmittel. Man reinigt durch Umkristallisation oder säulenchromatographisch über Kieselgel.

Verfahren C: 5.0 g des entsprechend mit R²O, R³ und R⁴ substituierten Oxiranylmethoxy-2H-1-benzopyran-2-ons werden mit einem Äquivalent des gewünschten mit R¹ substituierten Piperidins oder Piperazins in 250 ml Ethanol 1 bis 7 d unter Stickstoff bei 25 bis 50 °C gerührt. Man entfernt das Lösungsmittel im Vakuum und reinigt durch Umkristallisation oder säulenchromatographisch über Kieselgel.

Verfahren D: Man tropft bei Raumtemperatur unter Stickstoff zu 1.05 bis 1.1 Äquivalenten Natriumhydrid in Dimethylformamid ein Äquivalent des entsprechend mit R²O, R³ und R⁴ substituierten Hydroxy-2H-1-benzopyran-2-ons in Dimethylformamid und rührt zwei Stunden bei Raumtemperatur. Dazu gibt man 1.1 Äquivalente des gewünschten mit R¹ substituierten 1-(3-Chlorpropyl)piperidins oder -piperazins und etwa 0.2 Äquivalente Kaliumiodid und rührt 2.5 bis 5 h unter Stickstoff bei 85 bis 90°C. Man entfernt das Lösungsmittel im Vakuum, kristallisiert um oder nimmt in Chloroform auf, wäscht gegebenenfalls mit verdünnter Natronlauge, dann mit Wasser, trocknet über Natriumsulfat, entfernt erneut das Lösungsmittel und reinigt durch Umkristallisation.

Verfahren E: Zu einer Lösung der Base in Chloroform, Ethanol, Isopropanol, Aceton oder Chloroform/Diethylether gibt man ein bis zwei Äquivalente Fumarsäure, gelöst in Ethanol, Isopropanol oder Aceton. Das Fumarat fällt sofort oder beim Einengen kristallin an und wird umkristallisiert.

Verfahren F: In eine Lösung der Base in Diethylether und/oder Chloroform wird bis zur vollständigen Fällung des Hydrochlorids Chlorwasserstoffgas eingeleitet. Der entstandene Niederschlag wird abgesaugt und umkristallisiert.

Verfahren G: Zu einer Lösung der Base in Isopropanol, Chloroform oder Chloroform/Diethylether gibt man etwa 1.5 Äquivalente HCl (6 molar in Isopropanol). Der entstandene Niederschlag wird abgesaugt und umkristallisiert.

Bei den Verfahren E bis G wird, soweit in den Beispielen nichts anderes beschrieben wird, jeweils 1.0 HCl bzw. C₄H₄O₄ addiert.
Beispiel 1: 7-Methoxy-6-[3-(4-phenyl-1-piperazinyl)propoxy]-2H-1-benzopyran-2-on
   Verfahren A (23 h bei 60°C); Ausgangsmaterialien: 6-[3-(Methansulfonyloxy)propoxy]-7-methoxy-2H-1-benzopyran-2-on (Beispiel 67) und 1-Phenylpiperazin; Ausbeute 55 %; Schmp. 124 - 125°C (aus Ethanol).
   Fumarat: Verfahren E; Ausbeute 88 %; Schmp. 172 - 173°C (aus Ethanol).
Beispiel 2: 6-Methoxy-7-[3-(4-phenyl-1-piperazinyl)propoxy]-2H-1-benzopyran-2-on
   Verfahren B (9 h bei 70°C); Ausgangsmaterialien: 7-(3-Chlorpropoxy)-6-methoxy-2H-1-benzopyran-2-on (Beispiel 69) und 1-Phenylpiperazin; Ausbeute 94 %; Schmp. 82 - 83°C (aus Isopropanol/TBME).
   Fumarat: Verfahren E; Ausbeute 94 %; Schmp. 168 - 170°C (aus Isopropanol/TBME).
Beispiel 3: 7-Ethoxy-6-[3-(4-phenyl-1-piperazinyl)propoxy]-2H-1-benzopyran-2-on
   Verfahren A (26 h bei 60°C); Ausgangsmaterialien: 6-(3-Chlorpropoxy)-7-ethoxy-2H-1-benzopyran-2-on (Beispiel 70) und 1-Phenylpiperazin; Ausbeute 57 %; Schmp. 151 - 153°C (aus Methanol/Ethanol).
   Fumarat: Verfahren E; Ausbeute 78 %; Schmp. 170 - 173°C (aus Ethanol).
Beispiel 4: 6-Ethoxy-7-[3-(4-phenyl-1-piperazinyl)propoxy]-2H-1-benzopyran-2-on
   Verfahren B (40 h bei 50°C); Ausgangsmaterialien: 6-Ethoxy-7-[3-(methansulfonyloxy)propoxy]-2H-1-benzopyran-2-on (Beispiel 71) und 1-Phenylpiperazin; Ausbeute 76 %; Schmp. 116 - 117°C (aus Isopropanol).
   Fumarat: Verfahren E; Ausbeute 94 %; Schmp. 185 - 187°C (aus Ethanol/Aceton).
Beispiel 5: 6-(Cyclopentyloxy)-7-[3-(4-phenyl-1-piperazinyl)-propoxy]-2H-1-benzopyran-2-on
   Verfahren B (25 h bei 40°C); Ausgangsmaterialien: 6-(Cyclopentyloxy)-7-[3-(methansulfonyloxy)propoxy]-2H-1-benzopyran-2-on (Beispiel 72) und 1-Phenylpiperazin; Ausbeute 68 %; Schmp. 101 - 103°C (aus TBME/Isopropanol).
   Fumarat: Verfahren E; Ausbeute 88 %; Schmp. 171 - 173°C (aus Aceton).
Beispiel 6: 7-Methoxy-6-{3-[4-(3-trifluormethylphenyl)-1-piperazinyl]propoxy}-2H-1-benzopyran-2-on
   Verfahren A (10 h bei 80°C); Ausgangsmaterialien: 6-[3-(Methansulfonyloxy)propoxy]-7-methoxy-2H-1-benzopyran-2-on (Beispiel 67) und 1-(3-Trifluormethylphenyl)piperazin; Ausbeute 71 % Öl.
   Hydrochlorid: Verfahren F; Ausbeute 87 %; Schmp. 120 - 122°C (aus Ethanol).
Beispiel 7: 7-Ethoxy-6-{3-[4-(2-methoxyphenyl)-1-piperazinyl]propoxy}-2H-1-benzopyran-2-on
   Verfahren A (48 h bei 60°C); Ausgangsmaterialien: 6-(3-Chlorpropoxy)-7-ethoxy-2H-1-benzopyran-2-on (Beispiel 70) und 1-(2-Methoxyphenyl)piperazin Hydrochlorid; Ausbeute 49 %; Schmp. 78 - 80°C (aus Isopropanol).
   Fümarat: Verfahren E; Ausbeute 82 %; Schmp. 149 - 150°C (aus Ethanol).
Beispiel 8: 7-{3-[4-(Bis(4-fluorphenyl)methyl)-1-piperazinyl]propoxy}-6-methoxy-2H-1-benzopyran-2-on
   Verfahren B (36 h bei 60°C); Ausgangsmaterialien: 7-[3-(Methansulfonyloxy)propoxy]-6-methoxy-2H-1-benzopyran-2-on (Beispiel 68) und 1-[Bis(4-fluorphenyl)methyl]piperazin; Aus beute 76 % Öl.
   Fumarat: Verfahren E; Ausbeute 82 % ; Schmp. 200 - 202°C (aus Aceton).
Beispiel 9: 7-Methoxy-6-[3-(4-phenyl-1-piperidinyl)propoxy]-2H-1-benzopyran-2-on
   Verfahren A (4 h bei 80°C); Ausgangsmaterialien: 6-[3-(Methansulfonyloxy)propoxy]-7-methoxy-2H-1-benzopyran-2-on (Beispiel 67) und 4-Phenylpiperidin; Ausbeute 55 %; Schmp. 123 - 125°C (aus Isopropanol).
   Hydrochlorid: Verfahren G; Ausbeute 96 %; Schmp. 212 - 215°C (aus Isopropanol/Ethanol).
Beispiel 10: 6-Methoxy-7-[3-(4-phenyl-1-piperidinyl)propoxy]-2H-1-benzopyran-2-on
   Verfahren B (20 h bei 60°C); Ausgangsmaterialien: 7-[3-(Methansulfonyloxy)propoxy]-6-methoxy-2H-1-benzopyran-2-on (Beispiel 68) und 4-Phenylpiperidin; Ausbeute 73 %; Schmp. 97 - 99°C (aus Isopropanol).
   Hydrochlorid: Verfahren G; Ausbeute 93 %; Schmp. 198 - 200°C (aus Isopropanol).
Beispiel 11: 6-Ethoxy-7-[3-(4-phenyl-1-piperidinyl)propoxy]-2H-1-benzopyran-2-on
   Verfahren B (40 h bei 50°C); Ausgangsmaterialien: 6-Ethoxy-7-[3-(methansulfonyloxy)propoxy]-2H-1-benzopyran-2-on (Beispiel 71) und 4-Phenylpiperidin; Ausbeute 81 %; Schmp. 77 - 79°C (aus TBME/Isopropanol).
   Fumarat: Verfahren E; Ausbeute 74 %; Schmp. 212 - 215°C (aus Ethanol/Aceton).
Beispiel 12: 6-(1-Methylethoxy)-7-[3-(4-phenyl-1-piperidinyl)propoxy]-2H-1-benzopyran-2-on
   Verfahren B (24 h bei 60°C); Ausgangsmaterialien: 7-[3-(Methansulfonyloxy)propoxy]-6-(1-methylethoxy)-2H-1-benzopyran-2-on (Beispiel 73) und 4-Phenylpiperidin; Ausbeute 69 %; Schmp. 86 - 87°C (aus TBME).
   Fumarat: Verfahren E; Ausbeute 90 % ; Schmp. 164 - 167°C (aus Ethanol/Aceton).
Beispiel 13: 6-(Cyclopentyloxy)-7-[3-(4-phenyl-1-piperidinyl)propoxy]-2H-1-benzopyran-2-on
   Verfahren B (30 h bei 45°C); Ausgangsmaterialien: 6-(Cyclopentyloxy)-7-[3-(methansulfonyloxy)propoxy]-2H-1-benzopyran-2-on (Beispiel 72) und 4-Phenylpiperidin; Ausbeute 48 %; Schmp. 96 - 97°C (aus TBME/Isopropanol).
   Fumarat: Verfahren E; Ausbeute 83 %; Schmp. 171 - 173°C (aus Aceton).
Beispiel 14: 7-{3-[4-(Bis(4-fluorphenyl)hydroxymethyl)-1-piperidinyl]propoxy}-6-methoxy-2H-1-benzopyran-2-on
   Verfahren B (36 h bei 60°C); Ausgangsmaterialien: 7-[3-(Methansulfonyloxy)propoxy]-6-methoxy-2H-1-benzopyran-2-on (Beispiel 68) und 4-[Bis(4-fluorphenyl)hydroxymethyl]piperidin; Ausbeute 46 % Öl.
   Fumarat (x 0.5 C₄H₄O₄): Verfahren E; Ausbeute 60 %; Schmp. 140 - 142°C (aus Aceton/Petrolether).
Beispiel 15: 6-{3-[4-(Bis(4-fluorphenyl)methylen)-1-piperidinyl]propoxy}-7-methoxy-2H-1-benzopyran-2-on
   Verfahren A (6 h bei 80°C); Ausgangsmaterialien: 6-[3-(Methansulfonyloxy)propoxy]-7-methoxy-2H-1-benzopyran-2-on (Beispiel 67) und 4-[Bis-(4-fluorphenyl)hydroxymethyl]piperidin; Aus beute 64 % Öl. Erwärmen in alkoholischer Salzsäure führt unter Abspaltung von Wasser zur Titelverbindung.
   Fumarat: Verfahren E; Ausbeute 79 % ; Schmp. 135 - 138°C (aus Ethanol/Petrolether).
Beispiel 16: 6-{3-[4-(Diphenylmethyl)-1-piperidinyl]propoxy}-7-methoxy-2H-1-benzopyran-2-on
   Verfahren B (6 h bei 80°C); Ausgangsmaterialien: 6-[3-(Methansulfonyloxy)propoxy]-7-methoxy-2H-1-benzopyran-2-on (Beispiel 67) und 4-(Diphenylmethyl)piperidin; Ausbeute 75 % Öl.
   Fumarat: Verfahren E; Ausbeute 73 %; Schmp. 199 - 200°C (aus Aceton/Methanol).
Beispiel 17: 7-{3-[4-(Diphenylhydroxymethyl)-1-piperidinyl]-propoxy}-6-methoxy-2H-1-benzopyran-2-on
   Verfahren B (12 h bei 70°C); Ausgangsmaterialien: 7-(3-Chlorpropoxy)-6-methoxy-2H-1-benzopyran-2-on (Beispiel 69) und 4-(Diphenylhydroxymethyl)piperidin; Ausbeute 54 % Öl.
   Fumarat: Verfahren E; Ausbeute 82 %; Schmp. 155 - 157°C (aus Ethanol/Wasser).
Beispiel 18: (+-)-7-Methoxy-6-[2-hydroxy-3-(4-phenyl-1-piperazinyl)propoxy]-2H-1-benzopyran-2-on
   Verfahren C (5 d bei 40°C); Ausgangsmaterialien: 7-Methoxy-6-(oxiranylmethoxy)-2H-1-benzopyran-2-on und 1-Phenylpiperazin; Ausbeute 99 %; Schmp. 160 - 162°C (aus Isopropanol/Ethanol).
   Hydrochlorid: Verfahren F; Ausbeute 78 %; Schmp. 222 - 225°C (aus Isopropanol/Ethanol/Methanol).
Beispiel 19: (+-)-6-{3-[4-(3-Chlorphenyl)-1-piperazinyl]-2-hydroxypropoxy}-7-methoxy-2H-1-benzopyran-2-on
   Verfahren C (3 d bei 40°C); Ausgangsmaterialien: 7-Methoxy-6-(oxiranylmethoxy)-2H-1-benzopyran-2-on und 1-(3-Chlorphenyl)-piperazin; Ausbeute 85 %; Schmp. 140 - 141°C (aus Isopropanol).
   Hydrochlorid (x 1.7 HCl): Verfahren F; Ausbeute 88 %; Schmp. 222 - 224°C.
Beispiel 20: (+-)-6-{2-Hydroxy-3-[4-(3-trifluormethylphenyl)-1-piperazinyl]propoxy}-7-methoxy-2H-1-benzopyran-2-on
   Verfahren C (7 d bei 25°C); Ausgangsmaterialien: 7-Methoxy-6-(oxiranylmethoxy)-2H-1-benzopyran-2-on und 1-(3-Trifluormethylphenyl)piperazin; wird roh zum Hydrochlorid umgesetzt.
   Hydrochlorid: Verfahren F; Ausbeute 30 % (bezogen auf das Oxiran); Schmp. 196 - 197°C (aus Isopropanol).
Beispiel 21: (+-)-7-{2-Hydroxy-3-[4-(3-trifluormethylphenyl)-1-piperazinyl]propoxy}-6-methoxy-2H-1-benzopyran-2-on
   Verfahren C (1.5 d bei 45°C); Ausgangsmaterialien: 6-Methoxy-7-(oxiranylmethoxy)-2H-1-benzopyran-2-on (Beispiel 86) und 1-(3-Trifluormethylphenyl)piperazin; Ausbeute 80 %; Schmp. 133 - 135°C (aus Methanol).
   Hydrochlorid: Verfahren F; Ausbeute 83 %; Schmp. 125 - 128°C (Zers.; aus Isopropanol).
Beispiel 22: (+-)-6-[2-Hydroxy-3-(4-phenyl-1-piperidinyl)-propoxy]-7-methoxy-2H-1-benzopyran-2-on
   Verfahren C (1 d bei 50°C); Ausgangsmaterialien: 7-Methoxy-6-(oxiranylmethoxy)-2H-1-benzopyran-2-on und 4-Phenylpiperidin; Ausbeute 88 % Öl.
   Hydrochlorid: Verfahren F; Ausbeute 50 %; Schmp. 213 - 216°C (aus Isopropanol/Ethanol).
Beispiel 23: (+-)-7-[2-Hydroxy-3-(4-phenyl-1-piperidinyl)-propoxy]-6-methoxy-2H-1-benzopyran-2-on
   Verfahren C (27 h bei 45°C); Ausgangsmaterialien: 6-Methoxy-7-(oxiranylmethoxy)-2H-1-benzopyran-2-on (Beispiel 86) und 4-Phenylpiperidin; Ausbeute 69 %; Schmp. 114 - 115°C (aus Isopropanol/Ethanol).
   Hydrochlorid: Verfahren G; Ausbeute 82 %; Schmp. 202 - 205°C (aus Isopropanol/Ethanol).
Beispiel 24: (+-)-6-[2-Hydroxy-3-(4-hydroxy-4-phenyl-1-piperidinyl)propoxy]-7-methoxy-2H-1-benzopyran-2-on
   Verfahren C (3 d bei 40°C); Ausgangsmaterialien: 7-Methoxy-6-(oxiranylmethoxy)-2H-1-benzopyran-2-on und 4-Hydroxy-4-phenylpiperidin; Ausbeute 69 %; Schmp. 155 - 157°C (aus Isopropanol).
   Hydrochlorid: Verfahren G; Ausbeute 88 %; Schmp. 202 - 203°C (aus Isopropanol).
Beispiel 25: (+-)-6-{3-[4-(Bis(4-fluorphenyl)hydroxymethyl)-1-piperidinyl]-2-hydroxypropoxy}-7-methoxy-2H-1-benzopyran-2-on
   Verfahren C (5 d bei 45°C); Ausgangsmaterialien: 7-Methoxy-6-(oxiranylmethoxy)-2H-1-benzopyran-2-on und 4-[Bis(4-fluorphenyl)hydroxymethyl]piperidin; Ausbeute 45 %; Schmp. 220 - 221°C (aus Methanol/Chloroform).
   Hydrochlorid: Verfahren G; Ausbeute 99 %; Schmp. 172 - 174°C (aus Isopropanol/Ethanol).
Beispiel 26: 7-Methoxy-4-methyl-6-[3-(4-phenyl-1-piperazinyl)propoxy]-2H-1-benzopyran-2-on
   Verfahren B (32 h bei 50°C); Ausgangsmaterialien: 6-[3-(Methansulfonyloxy)propoxy]-7-methoxy-4-methyl-2H-1-benzopyran-2-on (Beispiel 74) und 1-Phenylpiperazin; Ausbeute 77 %; Schmp. 137 - 140°C (aus Isopropanol/Ethanol).
   Fumarat: Verfahren E; Ausbeute 79 %; Schmp. 195 - 200°C (aus Ethanol/Aceton).
Beispiel 27: 7-Methoxy-4-methyl-6-[3-(4-phenyl-1-piperidinyl)propoxy]-2H-1-benzopyran-2-on
   Verfahren B (26 h bei 50°C); Ausgangsmaterialien: 6-[3-(Methansulfonyloxy)propoxy]-7-methoxy-4-methyl-2H-1-benzopyran-2-on (Beispiel 74) und 4-Phenylpiperidin; Ausbeute 77 %; Schmp. 141 - 143°C (aus Isopropanol/Ethanol).
   Fumarat: Verfahren E; Ausbeute 89 %; Schmp. 221 - 223°C (aus Ethanol).
Beispiel 28: 7-Methoxy-3,4-dimethyl-6-[3-(4-phenyl-1-piperazinyl)propoxy]-2H-1-benzopyran-2-on
   Verfahren A (45 h bei 50°C); Ausgangsmaterialien: 6-[3-(Methansulfonyloxy)propoxy]-7-methoxy-3,4-dimethyl-2H-1-benzopyran-2-on (Beispiel 75) und 1-Phenylpiperazin; Ausbeute 64 %; Schmp. 151 - 152°C (aus Ethanol/Methanol).
   Fumarat (x 0.5 C₄H₄O₄): Verfahren E; Ausbeute 92 %; Schmp. 207 - 209°C (aus Ethanol/Methanol).
Beispiel 29: 7-Methoxy-3,4-dimethyl-6-[3-(4-phenyl-1-piperidinyl)propoxy]-2H-1-benzopyran-2-on
   Verfahren A (9 h bei 60°C); Ausgangsmaterialien: 6-[3-(Methansulfonyloxy)propoxy]-7-methoxy-3,4-dimethyl-2H-1-benzopyran-2-on (Beispiel 75) und 4-Phenylpiperidin; Ausbeute 64 %; Schmp. 152 - 153°C (aus Ethanol/Isopropanol).
   Fumarat (x 0.5 C₄H₄O₄): Verfahren E; Ausbeute 92 %; Schmp. 209 - 210°C (aus Ethanol/Methanol).
Beispiel 30: 6-{3-[4-(4-Chlorphenylmethyl)-1-piperazinyl]-propoxy}-7-methoxy-3,4-dimethyl-2H-1-benzopyran-2-on
   Verfahren D (5 h bei 90°C); Ausgangsmaterialien: 6-Hydroxy-7-methoxy-3,4-dimethyl-2H-1-benzopyran-2-on (Beispiel 103) und 1-[(4-Chlorphenyl)methyl]-4-(3-chlorpropyl)piperazin; Aus beute: 74 %; Schmp. 96 - 97°C (aus Isopropanol/Petrolether).
   Hydrochlorid (x 2 HCl): Verfahren G; Ausbeute 95 %; Schmp. 255 - 260°C (aus Isopropanol/Wasser).
Beispiel 31: 7-Methoxy-6-[3-(4-phenyl-1-piperazinyl)propoxy]-4-(trifluormethyl)-2H-1-benzopyran-2-on
   Verfahren D (3 h bei 85°C); Ausgangsmaterialien: 6-Hydroxy-7-methoxy-4-(trifluormethyl)-2H-1-benzopyran-2-on (Beispiel 104) und 1-(3-Chlorpropyl)-4-phenylpiperazin; Ausbeute 37 %; Schmp. 115 - 116°C (aus Isopropanol).
   Fumarat: Verfahren E; Ausbeute 70 %; Schmp. 183 - 185°C (aus Ethanol/Isopropanol).
Beispiel 32: 7-Methoxy-6-[3-(4-phenyl-1-piperidinyl)propoxy]-4-(trifluormethyl)-2H-1-benzopyran-2-on
   Verfahren D (3 h bei 85°C); Ausgangsmaterialien: 6-Hydroxy-7-methoxy-4-(trifluormethyl)-2H-1-benzopyran-2-on (Beispiel 104) und 1-(3-Chlorpropyl)-4-phenylpiperidin; Ausbeute 45 %; Schmp. 125 - 126°C (aus TBME/Methanol).
   Fumarat: Verfahren E; Ausbeute 59 %; Schmp. 176 - 179°C (aus Ethanol/TBME).
Beispiel 33: 7-Methoxy-6-[3-(4-phenyl-1-piperazinyl)propoxy]-4-propyl-2H-1-benzopyran-2-on
   Verfahren A (21 h bei 60°C); Ausgangsmaterialien: 6-[3-(Methansulfonyloxy)propoxy]-7-methoxy-4-propyl-2H-1-benzopyran-2-on (Beispiel 76) und 1-Phenylpiperazin; Ausbeute 55 %; Schmp. 108 - 109°C (aus Isopropanol/Ethanol).
   Fumarat (x 1.2 C₄H₄O₄): Verfahren E; Ausbeute 79 %; Schmp. 206 - 207°C (aus Aceton/Methanol).
Beispiel 34: 7-Methoxy-6-[3-(4-phenyl-1-piperidinyl)propoxy]-4-propyl-2H-1-benzopyran-2-on
   Verfahren A (20 h bei 60°C); Ausgangsmaterialien: 6-[3-(Methansulfonyloxy)propoxy]-7-methoxy-4-propyl-2H-1-benzopyran-2-on (Beispiel 76) und 4-Phenylpiperidin; Ausbeute 54 %; Schmp. 101 - 102°C (aus TBME/Petrolether).
   Fumarat: Verfahren E; Ausbeute 62 %; Schmp. 191 - 194°C (aus Aceton/Methanol).
Beispiel 35: 7-Methoxy-4-(1-methylethyl)-6-[3-(4-phenyl-1-piperazinyl)propoxy]-2H-1-benzopyran-2-on
   4.0 g (17 mmol) 6-Hydroxy-7-methoxy-4-(1-methylethyl)-2H-1-benzopyran-2-on (Beispiel 105), 4.7 g (20.5 mmol) 1-(3-Chlorpropyl)-4-phenyl-piperazin, 4.7 g (34 mmol) Kaliumcarbonat und 1.0 g Kaliumiodid werden in 300 ml DMF 44 h unter Stickstoff bei 60 °C gerührt. Man filtriert, entfernt das Lösungsmittel im Vakuum, nimmt in Chloroform auf, wäscht mit verdünnter Natronlauge und mit Wasser, trocknet mit Natriumsulfat und dampft erneut ein. Man erhält 5.5 g (74 %) Kristalle mit Schmp. 94 - 96°C (aus Isopropanol/TBME).
   Fumarat: Verfahren E; Ausbeute 88 %; Schmp. 175 - 176°C (aus Isopropanol/Ethanol).
Beispiel 36: 7-Methoxy-4-(1-methylethyl)-6-[3-(4-phenyl-1-piperidinyl)propoxy]-2H-1-benzopyran-2-on
   Verfahren D (2.5 h bei 85°C); Ausgangsmaterialien: 6-Hydroxy-7-methoxy-4-(1-methylethyl)-2H-1-benzopyran-2-on (Beispiel 105) und 1-(3-Chlorpropyl)-4-phenyl-piperidin; Ausbeute 85 %; Schmp. 62 - 65°C (aus TBME).
   Fumarat: Verfahren E; Ausbeute 65 %; Schmp. 155 - 157°C (aus Aceton, ausgekocht mit TBME).
Beispiel 37: 3-Butyl-7-methoxy-4-methyl-6-[3-(4-phenyl-1-piperazinyl)propoxy]-2H-1-benzopyran-2-on
   Verfahren B (60 h bei 50°C); Ausgangsmaterialien: 3-Butyl-6-(3-chlorpropoxy)-7-methoxy-4-methyl-2H-1-benzopyran-2-on (Beispiel 77) und 1-Phenylpiperazin; Ausbeute 72 %; Schmp. 130 - 131°C (aus Isopropanol/TBME).
   Fumarat: Verfahren E; Ausbeute 57 %; Schmp. 205 - 208°C (aus Aceton/Wasser).
Beispiel 38: 3-Butyl-7-methoxy-4-methyl-6-[3-(4-phenyl-1-piperidinyl)propoxy]-2H-1-benzopyran-2-on
   Verfahren B (60 h bei 50°C); Ausgangsmaterialien: 3-Butyl-6-(3-chlorpropoxy)-7-methoxy-4-methyl-2H-1-benzopyran-2-on (Beispiel 77) und 4-Phenylpiperidin; Ausbeute 51 % öliges Rohprodukt.
   Fumarat: Verfahren E; Ausbeute 89 %; Schmp. 176 - 178°C (aus Aceton).
Beispiel 39: 7-Methoxy-4-phenyl-6-[3-(4-phenyl-1-piperazinyl)propoxy]-2H-1-benzopyran-2-on
   Verfahren B (60 h bei 60°C); Ausgangsmaterialien: 6-[3-(Methansulfonyloxy)propoxy]-7-methoxy-4-phenyl-2H-1-benzopyran-2-on (Beispiel 78) und 1-Phenylpiperazin; Ausbeute 86 %; Schmp. 139 - 141°C (aus TBME/Isopropanol).
   Fumarat: Verfahren E; Ausbeute 93 %; Schmp. 145 - 147°C (aus Aceton/Diethylether).
Beispiel 40: 7-Methoxy-4-phenyl-6-[3-(4-phenyl-1-piperidinyl)propoxy]-2H-1-benzopyran-2-on
   Verfahren B (16 h bei 60°C); Ausgangsmaterialien: 6-[3-(Methansulfonyloxy)propoxy]-7-methoxy-4-phenyl-2H-1-benzopyran-2-on (Beispiel 78) und 4-Phenylpiperidin; Ausbeute 100 % Öl.
   Fumarat: Verfahren E; Ausbeute 84 %; Schmp. 150 - 152°C (aus Aceton).
Beispiel 41: 7-Methoxy-4-methyl-3-phenyl-6-[3-(4-phenyl-1-piperazinyl)propoxy]-2H-1-benzopyran-2-on
   Verfahren B (40 h bei 50°C); Ausgangsmaterialien: 6-(3-Chlorpropoxy)-7-methoxy-4-methyl-3-phenyl-2H-1-benzopyran-2-on (Beispiel 79) und 1-Phenylpiperazin; Ausbeute 66 %; Schmp. 168 - 170°C (aus Isopropanol).
   Fumarat: Verfahren E; Ausbeute 95 %; Schmp. 219 - 221°C (aus Aceton).
Beispiel 42: 7,8,9,10-Tetrahydro-3-methoxy-2-[3-(4-phenyl-1-piperazinyl)propoxy]-6H-dibenzo[b,d]pyran-6-on
   Verfahren B (24 h bei 60°C); Ausgangsmaterialien: 7,8,9,10-Tetrahydro-2-[3-(methansulfonyloxy)propoxy]-3-methoxy-6H-dibenzo[b,d]pyran-6-on (Beispiel 80) und 1-Phenylpiperazin; Ausbeute 94 %; Schmp. 104 - 106°C (aus TBME/ Isopropanol).
   Fumarat: Verfahren E; Ausbeute 92 %; Schmp. 160 - 161°C (aus Ethanol/Aceton).
Beispiel 43: 7,8,9,10-Tetrahydro-3-methoxy-2-[3-(4-phenyl-1-piperidinyl)propoxy]-6H-dibenzo[b,d]pyran-6-on
   Verfahren B (20 h bei 50°C); Ausgangsmaterialien: 7,8,9,10-Tetrahydro-2-[3-(methansulfonyloxy)propoxy]-3-methoxy-6H-dibenzo[b,d]pyran-6-on (Beispiel 80) und 4-Phenylpiperidin; Ausbeute 100 % (roh).
   Fumarat: Verfahren E; Ausbeute 72 % bezogen auf die Methansulfonylverbindung; Schmp. 184 - 186°C (aus Ethanol/Aceton).
Beispiel 44: 2,3-Dihydro-7-methoxy-8-[2-(4-phenyl-1-piperazinyl)ethoxy]cyclopenta[c][1]benzopyran-4(1H)-on
   Verfahren B (24 h bei 80°C); Ausgangsmaterialien: 2,3-Dihydro-8-[2-(methansulfonyloxy)ethoxy]-7-methoxy-cyclopenta[c][1]-benzopyran-4(1H)-on (Beispiel 81) und 1-Phenylpiperazin; Ausbeute 90 %; Schmp. 155 - 156°C (aus Isopropanol).
   Fumarat: Verfahren E; Ausbeute 94 %; Schmp. 182 - 185°C (aus Aceton).
Beispiel 45: 2,3-Dihydro-7-methoxy-8-[3-(4-phenyl-1-piperazinyl)propoxy]cyclopenta[c][1]benzopyran-4(1H)-on
   Verfahren B (40 h bei 50°C); Ausgangsmaterialien: 2,3-Dihydro-8-[3-(methansulfonyloxy)propoxy]-7-methoxy-cyclopenta[c][1]-benzopyran-4(1H)-on (Beispiel 82) und 1-Phenylpiperazin; Ausbeute > 100 % öliges Rohprodukt.
   Fumarat: Verfahren E; Ausbeute 72 % bezogen auf die Methansulfonylverbindung; Schmp. 184 - 186°C (aus Ethanol/Aceton).
Beispiel 46: 2,3-Dihydro-7-methoxy-8-[4-(4-phenyl-1-piperazinyl)butyloxy]cyclopenta[c][1]benzopyran-4(1H)-on
   Verfahren B (16 h bei 80°C); Ausgangsmaterialien: 2,3-Dihydro-8-[4-(methansulfonyloxy)butyloxy]-7-methoxy-cyclopenta[c][1]-benzopyran-4(1H)-on (Beispiel 84) und 1-Phenylpiperazin; Ausbeute 55 %; Schmp. 162 - 165°C (aus Ethanol).
   Fumarat: Verfahren E; Ausbeute 95 %; Schmp. 179 - 183°C (aus Aceton).
Beispiel 47: 2,3-Dihydro-7-methoxy-8-[5-(4-phenyl-1-piperazinyl)pentyloxy]cyclopenta[c][1]benzopyran-4(1H)-on
   Verfahren B (10 h bei 80°C); Ausgangsmaterialien: 8-(5-Brompentyloxy)-2,3-dihydro-7-methoxy-cyclopenta[c][1]benzopyran-4(1H)-on (Beispiel 85) und 1-Phenylpiperazin; Ausbeute 97 %; Schmp. 118 - 120°C (aus Isopropanol).
   Fumarat (x 0.5 C4H404): Verfahren E; Ausbeute 93 %; Schmp. 153 - 155°C (aus Isopropanol/TBME).
Beispiel 48: 2,3-Dihydro-7-methoxy-8-[2-(4-phenyl-1-piperidinyl)ethoxy]cyclopenta[c][1]benzopyran-4(1H)-on
   Verfahren B (16 h bei 60°C); Ausgangsmaterialien: 2,3-Dihydro-8-[2-(methansulfonyloxy)ethoxy]-7-methoxy-cyclopenta[c][1]-benzopyran-4(1H)-on (Beispiel 81) und 4-Phenylpiperidin; Ausbeute 93 %; Schmp. 150 - 152°C (aus Isopropanol).
   Fumarat: Verfahren E; Ausbeute 84 %; Schmp. 185 - 187°C (aus Ethanol/Isopropanol).
Beispiel 49: 2,3-Dihydro-7-methoxy-8-[3-(4-phenyl-1-piperidinyl)propoxy]cyclopenta[c][1]benzopyran-4(1H)-on
   Verfahren B (60 h bei 25°C); Ausgangsmaterialien: 2,3-Dihydro-8-[3-(methansulfonyloxy)propoxy]-7-methoxy-cyclopenta[c][1]-benzopyran-4(1H)-on (Beispiel 82) und 4-Phenylpiperidin; Ausbeute 65 %; Schmp. 164 - 166°C (aus Isopropanol).
   Fumarat: Verfahren E; Ausbeute 86 %; Schmp. 195 - 198°C (aus Ethanol).
Beispiel 50: 2,3-Dihydro-7-methoxy-8-[4-(4-phenyl-1-piperidinyl)butyloxy]cyclopenta[c][1]benzopyran-4(1H)-on
   Verfahren B (16 h bei 60°C); Ausgangsmaterialien: 2,3-Dihydro-8-[4-(methansulfonyloxy)butyloxy]-7-methoxy-cyclopenta[c][1]-benzopyran-4(1H)-on (Beispiel 84) und 4-Phenylpiperidin; Ausbeute 73 %; Schmp. 104 - 105°C (aus TBME).
   Fumarat: Verfahren E; Ausbeute 77 %; Schmp. 192 - 193°C (aus Isopropanol).
Beispiel 51: 2,3-Dihydro-7-methoxy-8-[5-(4-phenyl-1-piperidinyl)pentyloxy]cyclopenta[c][1]benzopyran-4(1H)-on
   Verfahren B (20 h bei 80°C); Ausgangsmaterialien: 8-(5-Brompentyloxy)-2,3-dihydro-7-methoxy-cyclopenta[c][1]benzopyran-4(1H)-on (Beispiel 85) und 4-Phenylpiperidin; Ausbeute 37 %; Schmp. 103 - 105°C (aus Isopropanol/TBME).
   Fumarat: Verfahren E; Ausbeute 94 %; Schmp. 176 - 178°C (aus Isopropanol/TBME).
Beispiel 52: 8-{3-[4-(4-Fluorphenyl)-1-piperazinyl]propoxy}-2,3-dihydro-7-methoxy-cyclopenta[c][1]benzopyran-4(1H)-on
   Verfahren B (12 h bei 55°C); Ausgangsmaterialien: 2,3-Dihydro-8-[3-(methansulfonyloxy)propoxy]-7-methoxy-cyclopenta[c][1]-benzopyran-4(1H)-on (Beispiel 82) und 1-(4-Fluorphenyl)piperazin; Ausbeute 84 %; Schmp. 139 - 141°C (aus Ethanol).
   Fumarat: Verfahren E; Ausbeute 90 %; Schmp. 195 - 200°C (aus Ethanol).
Beispiel 53: 8-{3-[4-(2,3-Dimethylphenyl)-1-piperazinyl]propoxy}-2,3-dihydro-7-methoxy-cyclopenta[c][1]benzopyran-4(1H)-on
   Verfahren B (20 h bei 60°C); Ausgangsmaterialien: 2,3-Dihydro-8-[3-(methansulfonyloxy)propoxy]-7-methoxy-cyclopenta[c][1]-benzopyran-4(1H)-on (Beispiel 82) und 1-(2,3-Dimethylphenyl)-piperazin; Ausbeute 84 %; Schmp. 164 - 166°C (aus Ethanol).
   Fumarat: Verfahren E; Ausbeute 94 %; Schmp. 235 - 237°C (aus Aceton).
Beispiel 54: 2,3-Dihydro-7-methoxy-8-{3-[4-(2-methoxyphenyl)-1-piperazinyl]propoxy}cyclopenta[c][1]benzopyran-4(1H)-on
   Verfahren A (40 h bei 50°C); Ausgangsmaterialien: 8-(3-Chlorpropoxy)-2,3-dihydro-7-methoxy-cyclopenta[c][1]benzopyran-4(1H)-on (Beispiel 83) und 1-(2-Methoxyphenyl)piperazin Hydrochlorid; Ausbeute 56 %; Schmp. 177 - 178°C (aus Chloroform/ Ethanol).
   Fumarat: Verfahren E; Ausbeute 87 %; Schmp. 173 - 174°C (aus Aceton).
Beispiel 55: 2,3-Dihydro-7-methoxy-8-{3-[4-(3-methoxyphenyl)-1-piperazinyl]propoxy}cyclopenta[c][1]benzopyran-4(1H)-on
   Verfahren B (60 h bei 50°C); Ausgangsmaterialien: 8-(3-Chlorpropoxy)-2,3-dihydro-7-methoxy-cyclopenta[c][1]benzopyran-4(1H)-on (Beispiel 83) und 1-(3-Methoxyphenyl)piperazin; Ausbeute 58 %; Schmp. 130 - 132°C (aus Ethanol/ Isopropanol).
   Fumarat: Verfahren E; Ausbeute 80 %; Schmp. 174 - 175°C (aus Aceton).
Beispiel 56: 2,3-Dihydro-7-methoxy-8-{3-[4-(4-methoxyphenyl)-1-piperazinyl]propoxy}cyclopenta[c][1]benzopyran-4(1H)-on
   Verfahren A (60 h bei 40°C); Ausgangsmaterialien: 8-(3-Chlorpropoxy)-2,3-dihydro-7-methoxy-cyclopenta[c][1]benzopyran-4(1H)-on (Beispiel 83) und 1-(4-Methoxyphenyl)piperazin Dihydrochlorid; Ausbeute 45 %; Schmp. 155 - 156°C (aus Ethanol).
   Fumarat: Verfahren E; Ausbeute 96 %; Schmp. 207 - 210°C (aus Aceton).
Beispiel 57: 8-{3-[4-(2-Ethoxyphenyl)-1-piperazinyl]propoxy}-2,3-dihydro-7-methoxy-cyclopenta[c][1]benzopyran-4(1H)-on
   Verfahren B (40 h bei 50°C); Ausgangsmaterialien: 8-(3-Chlorpropoxy)-2,3-dihydro-7-methoxy-cyclopenta[c][1]benzopyran-4(1H)-on (Beispiel 83) und 1-(2-Ethoxyphenyl)piperazin Hydrochlorid; Ausbeute 72 %; Schmp. 169 - 170°C (aus Isopropanol).
   Fumarat: Verfahren E; Ausbeute 85 %; Schmp. 159 -161°C (aus Aceton).
Beispiel 58: 8-{3-[4-(2-Chlorphenyl)-1-piperazinyl]propoxy}-2,3-dihydro-7-methoxy-cyclopenta[c][1]benzopyran-4(1H)-on
   Verfahren B (70 h bei 50°C; 4 Äquivalente Kaliumhydrogencarbonat); Ausgangsmaterialien: 8-(3-Chlorpropoxy)-2,3-dihydro-7-methoxy-cyclopenta[c][1]benzopyran-4(1H)-on (Beispiel 83) und 1-(2-Chlorphenyl)piperazin Dihydrochlorid; Ausbeute 61 % Rohprodukt.
   Fumarat (x 0.5 C4H404): Verfahren E; Ausbeute 76 %; Schmp. 206 - 209°C (aus Aceton).
Beispiel 59: 2,3-Dihydro-7-methoxy-8-{3-[4-(2-methylphenyl)-1-piperazinyl]propoxy}cyclopenta[c][1]benzopyran-4(1H)-on
   Verfahren B (60 h bei 45°C; 4 Äquivalente Kaliumhydrogencarbonat); Ausgangsmaterialien: 8-(3-Chlorpropoxy)-2,3-dihydro-7-methoxy-cyclopenta[c][1]benzopyran-4(1H)-on (Beispiel 83) und 1-(2-Methylphenyl)piperazin Hydrochlorid; Ausbeute 52 % Rohprodukt.
   Fumarat: Verfahren E; Ausbeute 76 %; Schmp. 215 - 218°C (Zers.; aus Chloroform/Ethanol).
Beispiel 60: 2,3-Dihydro-8-{3-[4-(2-hydroxyphenyl)-1-piperazinyl]propoxy}-7-methoxy-cyclopenta[c][1]benzopyran-4(1H)-on
   Verfahren A (40 h bei 50°C; 4 Äquivalente Kaliumcarbonat); Ausgangsmaterialien: 8-(3-Chlorpropoxy)-2,3-dihydro-7-methoxycyclopenta[c][1]benzopyran-4(1H)-on (Beispiel 83) und 1-(2-Hydroxyphenyl)piperazin Dihydrobromid; Ausbeute 54 %; Schmp. 136 - 139°C (aus Chloroform/Ethanol).
   Fumarat: Verfahren E; Ausbeute 84 %; Schmp. 196 - 199°C (aus Aceton).
Beispiel 61: 2,3-Dihydro-7-methoxy-8-{3-[4-(4-Nitrophenyl)-1-piperazinyl]propoxy}cyclopenta[c][1]benzopyran-4(1H)-on
   Verfahren B (60 h bei 50°C); Ausgangsmaterialien: 8-(3-Chlorpropoxy)-2,3-dihydro-7-methoxy-cyclopenta[c][1]benzopyran-4(1H)-on (Beispiel 83) und 1-(4-Nitrophenyl)piperazin; Ausbeute 43 %; Schmp. 226 - 228°C (Zers.; aus Aceton).
   Fumarat: Verfahren E; Ausbeute 92 %; Schmp. 216°C (Zers.; aus Aceton).
Beispiel 62: 2,3-Dihydro-7-methoxy-8-{3-[4-(2-pyridinyl)-1-piperazinyl]propoxy}cyclopenta[c][1]benzopyran-4(1H)-on
   Verfahren B (75 h bei 45°C); Ausgangsmaterialien: 8-(3-Chlorpropoxy)-2,3-dihydro-7-methoxy-cyclopenta[c][1]benzopyran-4(1H)-on (Beispiel 83) und 1-(2-Pyridinyl)piperazin; Ausbeute 68 %; Schmp. 152 - 154°C (aus Chloroform/ Ethanol).
   Fumarat: Verfahren E; Ausbeute 96 %; Schmp. 205 - 207°C (aus Aceton).
Beispiel 63: 2,3-Dihydro-7-methoxy-8-{3-[4-(2-pyrimidinyl)-1-piperazinyl]propoxy}cyclopenta[c][1]benzopyran-4(1H)-on
   Verfahren B (60 h bei 50°C; 4 Äquivalente Kaliumhydrogencarbonat); Ausgangsmaterialien: 8-(3-Chlorpropoxy)-2,3-dihydro-7-methoxy-cyclopenta[c][1]benzopyran-4(1H)-on (Beispiel 83) und l-(2-Pyrimidinyl)piperazin Dihydrochlorid; Ausbeute 54 %; Schmp. 184 - 186°C (aus Chloroform/ Ethanol).
   Fumarat: Verfahren E; Ausbeute 77 %; Schmp. 226 - 228°C (aus Aceton).
Beispiel 64: 2,3-Dihydro-7-methoxy-8-{3-[4-(phenylmethyl)-1-piperidinyl]propoxy}cyclopenta[c][1]benzopyran-4(1H)-on
   Verfahren B (25 h bei 50°C); Ausgangsmaterialien: 2,3-Dihydro-8-[3-(methansulfonyloxy)propoxy]-7-methoxy-cyclopenta[c][1]-benzopyran-4(1H)-on (Beispiel 82) und 4-(Phenylmethyl)piperidin; Ausbeute 71 %; Schmp. 119 - 120°C (aus Ethanol).
   Fumarat: Verfahren E; Ausbeute 91 %; Schmp. 195 - 197°C (aus Aceton).
Beispiel 65: 8-{3-[4-(Bis(4-fluorphenyl)hydroxymethyl)-1-piperidinyl]propoxy}-2,3-dihydro-7-methoxy-cyclopenta[c][1]benzopyran-4(1H)-on
   Verfahren B (38 h bei 60°C); Ausgangsmaterialien: 8-(3-Chlorpropoxy)-2,3-dihydro-7-methoxy-cyclopenta[c][1]benzopyran-4(1H)-on (Beispiel 83) und 4-[Bis(4-fluorphenyl)hydroxymethyl]piperidin; Ausbeute 67 %; Schmp. 91°C (Zers.; aus Aceton/TBME).
   Fumarat (x 0.5 C₄H₄O₄): Verfahren E; Ausbeute 70 %; Schmp. 191°C (Zers.; aus Wasser).
Beispiel 66: 8-{3-[4-(2-Fluorphenyl)-1-piperazinyl]propoxy}-2,3-dihydro-7-methoxy-cyclopenta[c][1]benzopyran-4(1H)-on
   Verfahren B (50 h bei 60°C); Ausgangsmaterialien: 8-(3-Chlorpropoxy)-2,3-dihydro-7-methoxy-cyclopenta[c][1]benzopyran-4(1H)-on (Beispiel 83) und 1-(2-Fluorphenyl)piperazin; Ausbeute 59 %; Schmp. 148 - 150°C (aus Ethanol).
   Fumarat (x H₂O): Verfahren E; Ausbeute 78 %; Schmp. 205 - 210°C (aus Wasser).

### II. Beispiele 67 bis 85 für Zwischenprodukte der allgemeinen Formel III

Zur Herstellung der in den nachfolgenden Beispielen 67 bis 85 näher beschriebenen Verbindungen wurden folgende Verfahren benutzt:

Verfahren H: Das entsprechend mit R²O, R³ und R⁴ substituierte Hydroxyalkoxy-2H-1-benzopyran-2-on und 1.5 bis 3 Äquivalente Triethylamin werden in Chloroform bei 0 bis 25°C tropfenweise mit 1.5 bis 3 Äquivalenten Methansulfonsäurechlorid versetzt. Man rührt mindestens 30 min unter Stickstoff bei 0 bis 25°C, wäscht mit Wasser, trocknet über Natriumsulfat, dampft im Vakuum ein und reinigt durch Umkristallisation.

Verfahren I: Das entsprechend mit R²O, R³ und R⁴ substituierte Hydroxy-2H-1-benzopyran-2-on, 1.25 bis 1.5 Äquivalente des gewünschten α,ω-Dihalogenalkans und 1.5 Äquivalente Kaliumcarbonat werden in 2-Butanon unter Stickstoff 16 bis 60 h bei 80°C gerührt. Man filtriert, entfernt das Lösungsmittel und reinigt, wenn möglich, durch Umkristallisation. Gegebenenfalls nimmt man in Chloroform auf, wäscht mit Wasser, trocknet über Natriumsulfat, dampft im Vakuum ein und reinigt dann durch Umkristallisation.
Beispiel 67: 6-[3-(Methansulfonyloxy)propoxy]-7-methoxy-2H-1-benzopyran-2-on
   Verfahren H; Ausgangsmaterial: 6-(3-Hydroxypropoxy)-7-methoxy-2H-1-benzopyran-2-on (Beispiel 87); Ausbeute 91 %; Schmp. 136 - 137°C (aus Methanol/Ethanol).
Beispiel 68: 7-[3-(Methansulfonyloxy)propoxy]-6-methoxy-2H-1-benzopyran-2-on
   Verfahren H; Ausgangsmaterial: 7-(3-Hydroxypropoxy)-6-methoxy-2H-1-benzopyran-2-on (Beispiel 88); Ausbeute 90 %; Schmp. 148 - 149°C (aus Isopropanol/Ethanol).
Beispiel 69: 7-(3-Chlorpropoxy)-6-methoxy-2H-1-benzopyran-2-on
   Verfahren I; Ausgangsmaterialien: Scopoletin und 1-Brom-3-chlorpropan; Ausbeute 94 %; Schmp. 97 - 98°C (aus Ethanol/ Wasser).
Beispiel 70: 6-(3-Chlorpropoxy)-7-ethoxy-2H-1-benzopyran-2-on Verfahren I; Ausgangsmaterialien: 7-Ethoxy-6-hydroxy-2H-1-benzopyran-2-on (Beispiel 100) und l-Brom-3-chlorpropan; Ausbeute 91 %; Schmp. 91 - 92°C (aus Ethanol/ Wasser).
Beispiel 71: 6-Ethoxy-7-[3-(methansulfonyloxy)propoxy]-2H-1-benzopyran-2-on
   Verfahren H; Ausgangsmaterial: 6-Ethoxy-7-(3-hydroxypropoxy)-2H-1-benzopyran-2-on (Beispiel 89); Ausbeute 98 %; Schmp. 103 - 105°C (aus Ethanol).
Beispiel 72: 6-(Cyclopentyloxy)-7-[3-(methansulfonyloxy)propoxy]-2H-1-benzopyran-2-on
   Verfahren H; Ausgangsmaterial: 6-(Cyclopentyloxy)-7-(3-hydroxypropoxy)-2H-1-benzopyran-2-on (Beispiel 90); Ausbeute > 100 % öliges Rohprodukt.
Beispiel 73: 7-[3-(Methansulfonyloxy)propoxy]-6-(1-methylethoxy)-2H-1-benzopyran-2-on
   Verfahren H; Ausgangsmaterial: 7-(3-Hydroxypropoxy)-6-(1-methylethoxy)-2H-1-benzopyran-2-on (Beispiel 91); Aus beute 78 %; Schmp. 91 - 93°C (aus Ethanol).
Beispiel 74: 6-[3-(Methansulfonyloxy)propoxy]-7-methoxy-4-methyl-2H-1-benzopyran-2-on
   Verfahren H; Ausgangsmaterial: 6-(3-Hydroxypropoxy)-7-methoxy-4-methyl-2H-1-benzopyran-2-on (Beispiel 92); Ausbeute 94 %; Schmp. 158 - 159°C (aus Ethanol/Aceton).
Beispiel 75: 6-[3-(Methansulfonyloxy)propoxy]-7-methoxy-3,4-dimethyl-2H-1-benzopyran-2-on
   Verfahren H; Ausgangsmaterial: 6-(3-Hydroxypropoxy)-7-methoxy-3,4-dimethyl-2H-1-benzopyran-2-on (Beispiel 93); Ausbeute 85 %; Schmp. 163 - 164°C (aus Ethanol/ Methanol).
Beispiel 76: 6-[3-(Methansulfonyloxy)propoxy]-7-methoxy-4-propyl-2H-1-benzopyran-2-on
   Verfahren H; Ausgangsmaterial: 6-(3-Hydroxypropoxy)-7-methoxy-4-propyl-2H-1-benzopyran-2-on (Beispiel 94); Ausbeute 92 %; Schmp. 129 - 130°C (aus Methanol/Ethanol).
Beispiel 77: 3-Butyl-6-(3-chlorpropoxy)-7-methoxy-4-methyl-2H-1-benzopyran-2-on
   Verfahren I; Ausgangsmaterialien: 3-Butyl-6-hydroxy-7-methoxy-4-methyl-2H-1-benzopyran-2-on (Beispiel 107) und 1-Brom-3-chlorpropan; Ausbeute 99 %; Schmp. 106 - 107°C (aus Ethanol).
Beispiel 78: 6-[3-(Methansulfonyloxy)propoxy]-7-methoxy-4-phenyl-2H-1-benzopyran-2-on
   Verfahren H; Ausgangsmaterial: 6-(3-Hydroxypropoxy)-7-methoxy-4-phenyl-2H-1-benzopyran-2-on (Beispiel 95); Ausbeute 98 %; Schmp. 108 - 109°C (aus Ethanol).
Beispiel 79: 6-(3-Chlorpropoxy)-7-methoxy-4-methyl-3-phenyl-2H-1-benzopyran-2-on
   Verfahren I; Ausgangsmaterialien: 6-Hydroxy-7-methoxy-4-methyl-3-phenyl-2H-1-benzopyran-2-on (Beispiel 108) und 1-Brom-3-chlorpropan; Ausbeute 99 %; Schmp. 159 - 161°C (aus Ethanol).
Beispiel 80: 7,8,9,10-Tetrahydro-2-[3-(methansulfonyloxy)propoxy]-3-methoxy-6H-dibenzo[b,d]pyran-6-on
   Verfahren H; Ausgangsmaterial: 7,8,9,10-Tetrahydro-2-(3-hydroxypropoxy)-3-methoxy-6H-dibenzo[b,d]pyran-6-on (Beispiel 96). Ausbeute 87 %; Schmp. 139 - 141°C (aus Ethanol).
Beispiel 81: 2,3-Dihydro-8-[2-(methansulfonyloxy)ethoxy]-7-methoxy-cyclopenta[c][1]benzopyran-4(1H)-on
   Verfahren H; Ausgangsmaterial: 2,3-Dihydro-8-(2-hydroxyethoxy)-7-methoxy-cyclopenta[c][1]benzopyran-4(1H)-on (Beispiel 97); Ausbeute 96 %; Schmp. 185 - 190°C (aus Ethanol).
Beispiel 82: 2,3-Dihydro-8-[3-(methansulfonyloxy)propoxy]-7-methoxy-cyclopenta[c][1]benzopyran-4(1H)-on
   Verfahren H; Ausgangsmaterial: 2,3-Dihydro-8-(3-hydroxy-propoxy)-7-methoxy-cyclopenta[c][1]benzopyran-4(1H)-on (Bei spiel 98); Ausbeute 88 %; Schmp. 149 - 150°C (aus Ethanol).
Beispiel 83: 8-(3-Chlorpropoxy)-2,3-dihydro-7-methoxy-cyclopenta[c][1]benzopyran-4(1H)-on
   Verfahren I; Ausgangsmaterialien: 2,3-Dihydro-8-hydroxy-7-methoxy-cyclopenta[c][1]benzopyran-4(1H)-on (Beispiel 109) und 1-Brom-3-chlorpropan; Ausbeute 84 %; Schmp. 174 - 176°C (aus Ethanol).
Beispiel 84: 2,3-Dihydro-8-[4-(methansulfonyloxy)butyloxy]-7-methoxy-cyclopenta[c][1]benzopyran-4(1H)-on
   Verfahren H; Ausgangsmaterial: 2,3-Dihydro-8-(4-hydroxybutyloxy)-7-methoxy-cyclopenta[c][1]benzopyran-4(1H)-on (Beispiel 99); Ausbeute 91 %; Schmp. 130 - 132°C (aus Ethanol).
Beispiel 85: 8-(5-Brompentyloxy)-2,3-dihydro-7-methoxy-cyclopenta[c][1]benzopyran-4(1H)-on
   Verfahren I; Ausgangsmaterialien: 2,3-Dihydro-8-hydroxy-7-methoxy-cyclopenta[c][1]benzopyran-4(1H)-on (Beispiel 109) und 1,5-Dibrompentan; Ausbeute 51 %; Schmp. 103 - 104°C (aus TBME).

### III. Beispiel 86 für ein Zwischenprodukt der allgemeinen Formel IV

Beispiel 86: (+-)-6-Methoxy-7-(oxiranylmethoxy)-2H-1-benzopyran-2-on
25.0 g (130 mmol) Scopoletin, 15.3 ml (196 mmol) Epichlorhydrin, 36.0 g (260 mmol) Kaliumcarbonat und 2.5 g Kaliumiodid werden in 500 ml DMF unter Stickstoff 3 h bei 80°C gerührt. Man dampft im Vakuum ein, extrahiert mehrmals mit heißem Ethanol und dampft erneut ein: 21.2 g (66 %); Schmp. 143 - 145°C (aus Ethanol).

### IV. Beispiele 87 bis 99 für Zwischenprodukte der allgemeinen Formel XI mit Z = OH

Zur Herstellung der in den nachfolgenden Beispielen 87 bis 99 näher beschriebenen Verbindungen wurden folgende Verfahren benutzt:

Verfahren J: Das entsprechend mit R²O, R³ und R⁴ substituierte Hydroxy-2H-1-benzopyran-2-on, 2 bis 3 Äquivalente des gewünschten ω-Halogenalkanols, 2.5 bis 3 Äquivalente Kaliumcarbonat und 0.1 bis 0.2 Äquivalente Kaliumiodid werden in DMF 16 bis 45 h bei 60 bis 90°C gerührt. Man filtriert, dampft im Vakuum ein, nimmt in Chloroform auf, wäscht mit Wasser, trocknet über Natriumsulfat und dampft erneut ein.

Verfahren K: Man tropft das entsprechend mit R²O, R³ und R⁴ substituierte Hydroxy-2H-1-benzopyran-2-on, gelöst in DMF, bei 25°C unter Stickstoff in eine Suspension von 1.1 Äquivalenten Natriumhydrid in DMF und rührt mindestens 30 min. Dazu gibt man 1.5 bis 2 Äquivalente des gewünschten ω-Halogenalkanols und 0.1 bis 0.2 Äquivalente Kaliumiodid und rührt 7 bis 40 h bei 80 bis 90°C. Man dampft im Vakuum ein, nimmt in Chloroform auf, wäscht mit Wasser, trocknet über Natriumsulfat und dampft erneut ein.
Beispiel 87: 6-(3-Hydroxypropoxy)-7-methoxy-2H-1-benzopyran-2-on
   Verfahren J; Ausgangsmaterialien: Isoscopoletin und 3-Chlor-1-propanol; Ausbeute 69 %; Schmp. 126 - 127°C (aus TBME/ Isopropanol).
Beispiel 88: 7-(3-Hydroxypropoxy)-6-methoxy-2H-1-benzopyran-2-on
   Verfahren J; Ausgangsmaterialien: Scopoletin und 3-Chlor-1-propanol; Ausbeute 65 %; Schmp. 107 - 110°C (aus TBME).
Beispiel 89: 6-Ethoxy-7-(3-hydroxypropoxy)-2H-1-benzopyran-2-on
   Verfahren K; Ausgangsmaterialien: 6-Ethoxy-7-hydroxy-2H-1-benzopyran-2-on und 3-Chlor-1-propanol; Ausbeute 69 %; Schmp. 96 - 98°C (aus Isopropanol/TBME).
Beispiel 90: 6-(Cyclopentyloxy)-7-(3-hydroxypropoxy)-2H-1-benzopyran-2-on
   Verfahren K; Ausgangsmaterialien: 6-(Cyclopentyloxy)-7-hydroxy-2H-1-benzopyran-2-on (Beispiel 101) und 3-Chlor-1-propanol; Ausbeute 79 %; Schmp. 122 - 124°C (aus Isopropanol).
Beispiel 91: 7-(3-Hydroxypropoxy)-6-(1-methylethoxy)-2H-1-benzopyran-2-on
   Verfahren K; Ausgangsmaterialien: 7-Hydroxy-6-(1-methylethoxy)-2H-1-benzopyran-2-on (Beispiel 102) und 3-Chlor-1-propanol; Ausbeute 80 %; Schmp. 92 - 94°C (aus TBME).
Beispiel 92: 6-(3-Hydroxypropoxy)-7-methoxy-4-methyl-2H-1-benzopyran-2-on
   Verfahren K; Ausgangsmaterialien: 6-Hydroxy-7-methoxy-4-methyl-2H-1-benzopyran-2-on und 3-Chlor-1-propanol; Ausbeute 83 %; Schmp. 169 - 170.5°C (aus Isopropanol/Wasser).
Beispiel 93: 6-(3-Hydroxypropoxy)-7-methoxy-3,4-dimethyl-2H-1-benzopyran-2-on
   Verfahren K; Ausgangsmaterialien: 6-Hydroxy-7-methoxy-3,4-dimethyl-2H-1-benzopyran-2-on (Beispiel 103) und 3-Chlor-1-propanol; Ausbeute 82 %; Schmp. 160 - 161°C (aus Isopropanol/ Ethanol).
Beispiel 94: 6-(3-Hydroxypropoxy)-7-methoxy-4-propyl-2H-1-benzopyran-2-on
   Verfahren K; Ausgangsmaterialien: 6-Hydroxy-7-methoxy-4-propyl-2H-1-benzopyran-2-on (Beispiel 106) und 3-Chlor-1-propanol; Ausbeute 73 %; Schmp. 97 - 98°C (aus Isopropanol/ Ethanol).
Beispiel 95: 6-(3-Hydroxypropoxy)-7-methoxy-4-phenylbenzopyran-2-on
   Verfahren K; Ausgangsmaterialien: Dalbergin und 3-Chlor-1-propanol; Ausbeute 85 %; Schmp. 164 - 165°C (aus Isopropanol).
Beispiel 96: 7,8,9,10-Tetrahydro-2-(3-hydroxypropoxy)-3-methoxy-6H-dibenzo[b,d]pyran-6-on
   Verfahren K; Ausgangsmaterialien: 7,8,9,10-Tetrahydro-2-hydroxy-3-methoxy-6H-dibenzo[b,d]pyran-6-on (Beispiel 110) und 3-Chlor-1-propanol; Ausbeute 74 %; Schmp. 176 - 177°C (aus Isopropanol/Ethanol/Aceton).
Beispiel 97: 2,3-Dihydro-8-(2-hydroxyethoxy)-7-methoxy-cyclopenta[c][1]benzopyran-4(1H)-on
   Verfahren J; Ausgangsmaterialien: 2,3-Dihydro-8-hydroxy-7-methoxy-cyclopenta[c][1]benzopyran-4(1H)-on (Beispiel 109) und 2-Bromethanol; Ausbeute 95 %; Schmp. 159 - 160°C (aus Ethanol).
Beispiel 98: 2,3-Dihydro-8-(3-hydroxypropoxy)-7-methoxycyclopenta[c][1]benzopyran-4(1H)-on
   Verfahren J (jedoch Kaliumhydrogencarbonat); Ausgangsmaterialien: 2,3-Dihydro-8-hydroxy-7-methoxy-cyclopenta[c][1]benzopyran-4(1H)-on (Beispiel 109) und 3-Chlor-1-propanol; Ausbeute 58 %; Schmp. 213 - 215°C (aus Isopropanol).
Beispiel 99: 2,3-Dihydro-8-(4-hydroxybutyloxy)-7-methoxycyclopenta[c][1]benzopyran-4(1H)-on
   Verfahren J; Ausgangsmaterialien: 2,3-Dihydro-8-hydroxy-7-methoxy-cyclopenta[c][1]benzopyran-4(1H)-on (Beispiel 109) und 4-Chlor-1-butanol; Ausbeute 79 %; Schmp. 142 - 143°C (aus Ethylacetat/Petrolether).

### V. Beispiele 100 bis 110 für Zwischenprodukte der allgemeinen Formel VI

Beispiel 100: 7-Ethoxy-6-hydroxy-2H-1-benzopyran-2-on
   110 g (300 mmol) Esculin x 1.5 H20, 70 g (450 mmol) Ethyliodid und 82.8 g (600 mmol) Kaliumcarbonat werden in 400 ml DMF 30 h bei 85°C gerührt. Man filtriert, engt das Filtrat auf ca. 250 ml ein, gibt ca. 200 ml Ethanol dazu und läßt bei 0°C kristallisieren. Das Kristallisat wird in 800 ml 2n Schwefelsäure 5 h unter Rückfluß gekocht. Man saugt das Produkt ab, wäscht mit Wasser und kristallisiert aus Wasser/Ethanol um: 43.2 g (70 %), Schmp. 145 - 146°C.
Beispiel 101: 6-(Cyclopentyloxy)-7-hydroxy-2H-1-benzopyran-2-on
   50.0 g (186 mmol) 6-Hydroxy-7-phenylmethoxy-2H-1-benzopyran-2-on, 41.7 g (280 mmol) Cyclopentylbromid, 51.4 g (372 mmol) Kaliumcarbonat und 5.0 g Kaliumiodid werden in 500 ml DMF 18 h bei 80°C gerührt. Man filtriert, dampft das Filtrat ein, nimmt in Chloroform auf, wäscht mit Wasser, trocknet über Natriumsulfat und dampft erneut ein: 60.0 g (96%) 6-(Cyclopentyloxy)-7-(phenylmethoxy)-2H-1-benzopyran-2-on, Schmp. 140 - 141°C (aus Isopropanol). Davon werden 15.0 g (45 mmol) in 200 ml Ethanol in Gegenwart von 2.0 g Pd auf Aktivkohle (5 %) 3 h bei 25°C und 7 bar Wasserstoffdruck hydriert. Man filtriert und entfernt das Lösungsmittel: 9.0 g (82 %), Schmp. 154 - 155°C (aus Isopropanol/TBME).
Beispiel 102: 7-Hydroxy-6-(1-methylethoxy)-2H-1-benzopyran-2-on
   50.0 g (186 mmol) 6-Hydroxy-7-phenylmethoxy-2H-1-benzopyran-2-on, 36.3 ml (387 mmol) Isopropylbromid, 51.5 g (373 mmol) Kaliumcarbonat und 5.0 g Kaliumiodid werden in 500 ml DMF 14 h bei 80°C gerührt. Man filtriert, dampft das Filtrat ein und kristallisiert aus Isopropanol um: 57.7 g (100 %) 6-(1-Methylethoxy)-7-(phenylmethoxy)-2H-1-benzopyran-2-on. Davon werden 50.0 g (161 mmol) in 100 ml Eisessig und 90 ml konz. Salzsäure 2.5 h bei 70°C gerührt. Man dampft ein und reinigt säulen chromatographisch über Kieselgel (Eluens: Chloroform/Methanol 98/2 H 95/5): 24.65 g (69 %), Schmp. 121 - 122°C (aus Isopropanol).

Zur Herstellung der in den nachfolgenden Beispielen 103 bis 110 näher beschriebenen Verbindungen wurde folgendes Verfahren benutzt:

Verfahren L: Zu einer Lösung von Methoxyhydrochinon in Schwefelsäure (75 % in Wasser) tropft man bei 0 bis 25°C ein Äquivalent β-Ketoester X. Man rührt mindestens 30 min, gibt auf Eis und saugt das Produkt ab.
Beispiel 103: 6-Hydroxy-7-methoxy-3,4-dimethyl-2H-1-benzopyran-2-on
   Verfahren L; Ausgangsmaterial: 2-Methylacetessigsäureethylester; Ausbeute 79 %; Schmp. 226 - 229°C (aus Isopropanol/ Ethanol/Methanol).
Beispiel 104: 6-Hydroxy-7-methoxy-4-(trifluormethyl)-2H-1-benzopyran-2-on
   Verfahren L; Ausgangsmaterial: 4,4,4-Trifluoracetessigsäure ethylester; Ausbeute 13 %; Schmp. 190 - 192°C (aus TBME/ Petrolether).
Beispiel 105: 6-Hydroxy-7-methoxy-4-(1-methylethyl)-2H-1-benzopyran-2-on
   Verfahren L; Ausgangsmaterial: 4-Methyl-3-oxopentansäuremethylester; Ausbeute 39 %; Schmp. 126 - 127°C (aus TBME/ Isopropanol).
Beispiel 106: 6-Hydroxy-7-methoxy-4-propyl-2H-1-benzopyran-2-on
   Verfahren L; Ausgangsmaterial: 3-Oxohexansäureethylester; Ausbeute 68 %; Schmp. 163 - 164°C (aus Isopropanol/Ethanol).
Beispiel 107: 3-Butyl-6-hydroxy-7-methoxy-4-methyl-2H-1-benzopyran-2-on
   Verfahren L; Ausgangsmaterial: 2-Butylacetessigsäureethylester; Ausbeute 59 %; Schmp. 174 - 175°C (aus Ethanol).
Beispiel 108: 6-Hydroxy-7-methoxy-4-methyl-3-phenyl-2H-1-benzopyran-2-on
   Verfahren L; Ausgangsmaterial: 2-Phenylacetessigsäureethylester; Ausbeute 99 %; Schmp. 258 - 260°C (aus Ethanol).
Beispiel 109: 2,3-Dihydro-8-hydroxy-7-methoxy-cyclopenta[c]-[1]benzopyran-4(1H)-on
   Verfahren L; Ausgangsmaterial: Cyclopentanon-2-carbonsäureethylester; Ausbeute 71 %; Schmp. 254 - 255°C (aus Isopropanol).
Beispiel 110: 7,8,9,10-Tetrahydro-2-hydroxy-3-methoxy-6H-dibenzo[b,d]pyran-6-on
   Verfahren L; Ausgangsmaterial: Cyclohexanon-2-carbonsäureethylester; Ausbeute 86 %; Schmp. 184 - 186°C (aus Isopropanol).

### Pharmakologische Untersuchungen

Zur Ermittlung der neuroprotektiven/antikonvulsiven Aktivität der erfindungsgemäßen Verbindungen der allgemeinen Formel I wurden zwei leicht voneinander abweichende Methoden verwendet:
1. Zur Charakterisierung neuroprotektiver Wirksamkeit wurde männlichen NMRI-Mäusen mit einem Körpergewicht von 20-25 g NMDA in einer Menge von 25 mg/kg/10 ml intravenös appliziert. Als Folge dieser Applikation erleiden die Tiere klonische und teilweise auch tonische Konvulsionen, die zum Tode führen. Als Kriterium für die Wirksamkeit der untersuchten Verbindungen dient die Verhinderung des Todes.
2. Zur Charakterisierung antiepileptischer Wirksamkeit diente die gleiche Methode, wobei aber die NMDA-Dosis 40 mg/kg/10 ml i.v. betrug. Diese höhere NMDA-Dosis führte bei allen Versuchstieren zum Auftreten tonischer Konvulsionen.

Alle Versuchstiere hatten vor den Experimenten freien Zugang zu Futter und Wasser. Die Test- und Referenzsubstanzen wurden als Suspension in 0,2 % Agar oder Wasser, z.T. unter Zuhilfenahme von Lösungsvermittlern wie z.B. PEG-400, peroral durch Schlundsonde verabreicht. Kontrolltiere erhielten gleiche Volumina der Lösungsmittel gegebenenfalls unter Zusatz von Lösungsvermittlern appliziert. Eine Stunde nach Substanzapplikation wurde NMDA intravenös appliziert und das Auftreten von Konvulsionen beobachtet.

In Tabelle 1 sind die Ergebnisse der nach Methode 1 durchgeführten Tests (NMDA 25 mg i.v.) bei Dosen von 5-100 mg der erfindungsgemäßen Verbindungen pro kg Körpergewicht im Vergleich zu bekannten NMDA-Antagonisten aufgeführt. Als %-Wirkung ist der prozentuale Anteil der überlebenden Tiere angegeben.

In Tabelle 2 sind die Ergebnisse der nach Methode 2 durchgeführten Tests (NMDA 40 mg i.v.) bei Dosen von 5-25 mg der erfindungsgemäßen Verbindungen pro kg Körpergewicht im Vergleich zu bekannten Antikonvulsiva/Antiepileptika aufgeführt. Als %-Wirkung ist der prozentuale Anteil derjenigen Tiere angegeben, die bei dem Test vor dem Auftreten tonischer Konvulsionen geschützt waren.

In den Tabellen 1 und 2 sind außerdem als ED₅₀-Werte diejenigen Dosen der erfindungsgemäßen Substanzen und anderer NMDA-Antagonisten aufgeführt, die im NMDA-i.v.-Test (25 bzw. 40 mg/kg) eine Stunde nach Applikation 50 % der Tiere vor dem NMDA-induzierten Tod oder tonischen Konvulsionen schützen.

Die Bestimmung der ED₅₀-Werte erfolgte nach [Lichtfield und Wilcoxon, J. Pharmacol. exp. Therapeut. 96, 99 (1949)1 jeweils mit 4-5 Tiergruppen zu 10 Tieren je Dosisstufe.

Während der gesamten Versuchsdauer wurden die Tiere auf Anzeichen substanzbedingter Verhaltensänderungen und Neurotoxizität beobachtet. Bei sämtlichen erfindungsgemäßen Verbindungen wurden in den verwendeten Dosierungen keinerlei Anzeichen einer toxischen Eigenwirkung beobachtet. Bis zu einer Dosis von 200 mg/kg konnte keine Mortalität festgestellt werden.

Die Feststellung der antiallergischen und entzündungshemmenden Eigenschaften der erfindungsgemäßen Verbindungen erfolgte mit einem passiven Pfotenanaphylaxie-Modell bei der Ratte. Dieses pharmakologische Modell ist eine etablierte Standardmethode zur Untersuchung von Hypersensibilitätsreaktionen vom Typ I (Martel, R. und Klicius, J., Int. Archs. Allergy Appl. Immun. 55, 205-209 (1977), Nolan, J.C. et al., Agents and Actions 31, 210-218 (1990) und Walsh, D.A. et al., J. Med. Chem. 33, 1823-1827 (1990). Ähnlich wie bei allergischen Erkrankungen des Menschen sind auch an den passiven Anaphylaxiereaktionen bei der Ratte eine Vielzahl von Mediatoren beteiligt, wie z.B. Histamin, Serotonin, Leukotriene, Plättchen aktivierender Faktor etc. (Weg, V.B. et al., Eur. J. Pharmacol. 204, 157-163 (1991).

Für alle Versuche standen männliche Sprague-Dawley-Ratten zur Verfügung. Die Haltung der Versuchstiere erfolgte unter standardisierten Umweltbedingungen bei freiem Zugang zu Futter und Wasser. Zur Sensibilisierung gegenüber Antigen wurde den Versuchstieren 100 µl eines Maus-Antiserums gegen Ovalbumin subplantar in die linke Hinterpfote injiziert. Der Antikörper wurde dabei in einer Verdünnung verwendet, von der aus Versuchen bekannt war, daß sie ein signifikantes Pfotenödem auslöst. Die gegenüberliegende Pfote erhielt zur Kontrolle nur eine subplantare Injektion von 100 µl des Lösungsmittels (sterile physiologische NaCl-Lösung). Über Nacht wurde den Versuchstieren das Futter entzogen, Wasser stand ihnen aber weiterhin ad libitum zur Verfügung.

Am folgenden Morgen wurden die Versuchstiere unter Zuhilfenahme einer Schlundsonde peroral mit Test-, Referenz- oder Kontrollsubstanzen in 10 ml/kg 0,2 % Agar, dem teilweise Lösungsvermittler zugesetzt waren, behandelt. Pro Gruppe wurden bei allen Versuchen zwischen 6 und 8 Tiere verwendet. Eine Stunde später erfolgte die Auslösung der anaphylaktischen Reaktion durch die intravenöse Injektion von 10 mg/10 ml/kg Ovalbumin in physiologischer NaCl-Lösung. Nach dreißig Minuten wurden die Volumina beider Hinterpfoten plethysmographisch (Volumeter 2060, Rhema-Labortechnik, Hofheim) ermittelt. Das Ödemvolumen wurde aus der Differenz der Werte beider Pfoten errechnet. Alle ermittelten Werte sind in der Tabelle 3 zusammengefaßt. Die antiallergische Wirkung ist dabei als prozentuale Hemmung des Ödemvolumens von Tieren der Testgruppe gegenüber parallel untersuchten Kontrollen angegeben. In der Tabelle 3 sind zusätzlich Untersuchungsergebnisse bekannter Antiallergika aufgeführt. Die Bestimmung der ED₅₀-Werte erfolgte über eine lineare Regression nach Log-Transformation der verabreichten Dosen, wobei mindestens drei Dosisstufen verwenden wurden.

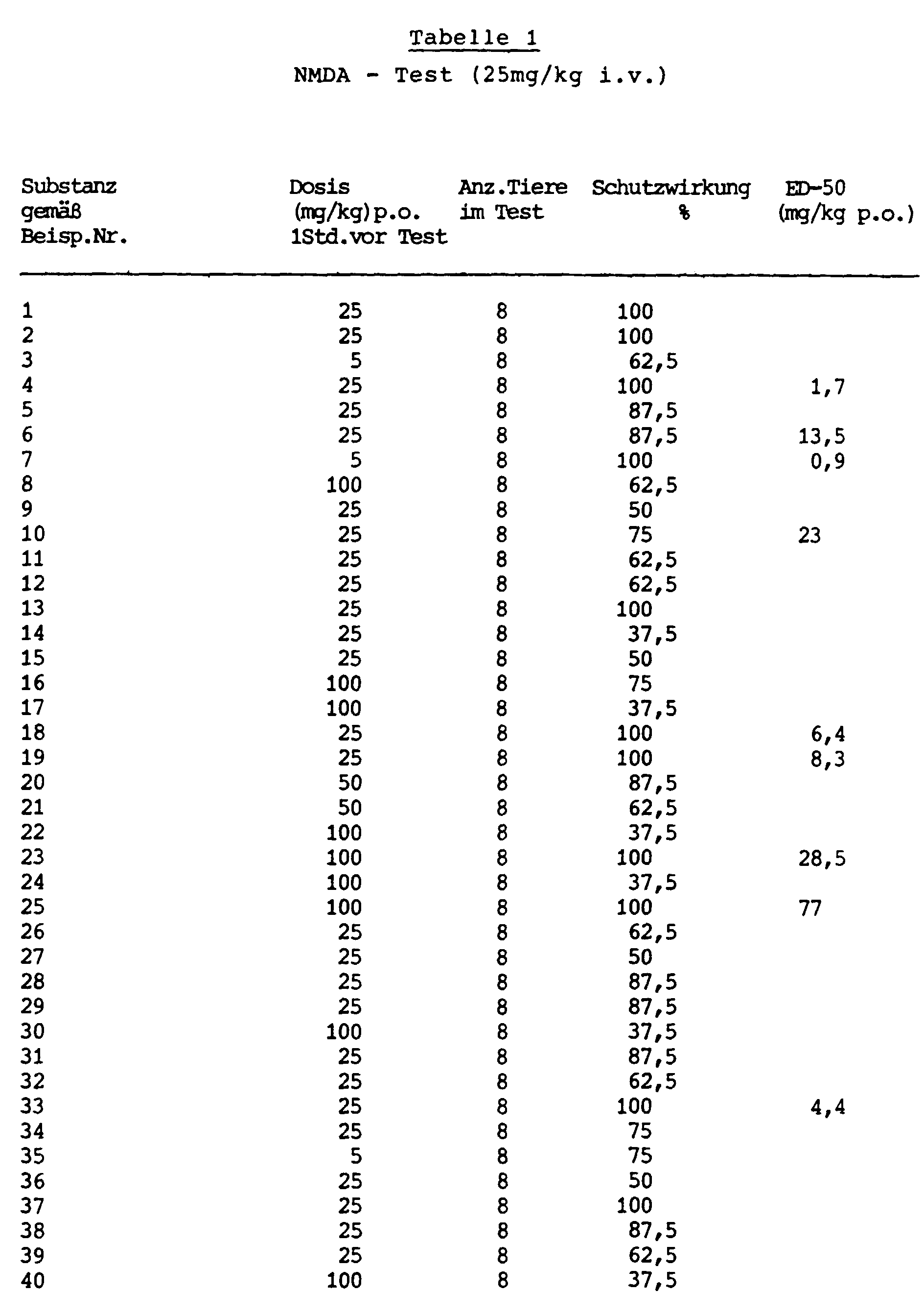

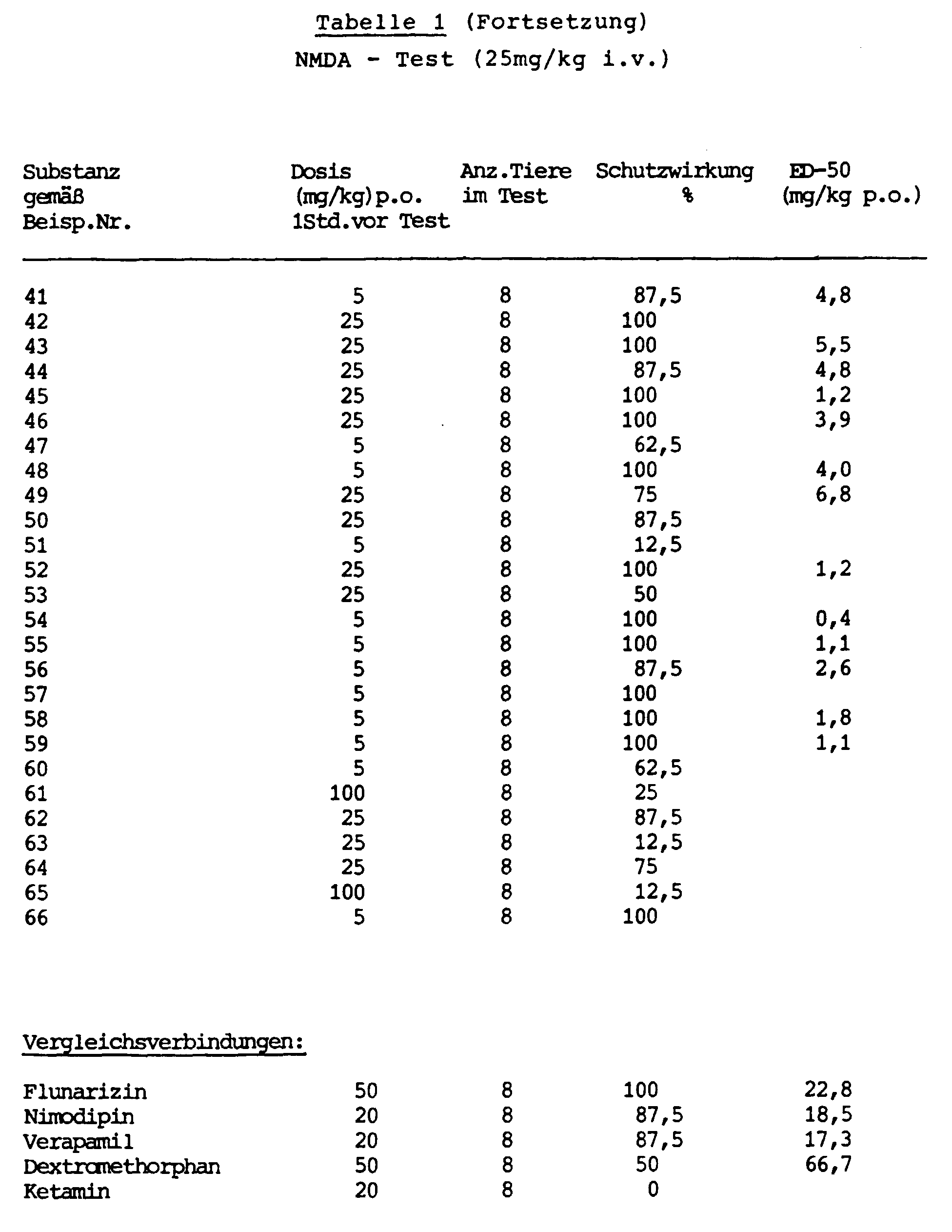

**Tabelle 2**

| NMDA - Test (40mg/kg i.v.) | | | | |
|---|---|---|---|---|
| Substanz gemäß Beisp.Nr. | Dosis (mg/kg)p.o. 1Std.vor Test | Anz.Tiere im Test | Schutzwirkung % | ED-50 (mg/kg p.o.) |
| 6 | 25 | 8 | 0 | |
| 10 | 5 | 8 | 25,0 | |
| 4 | 5 | 8 | 62,5 | |
| 49 | 20 | 8 | 87,5 | 4,0 |
| 43 | 5 | 8 | 37,5 | |
| 18 | 25 | 8 | 100 | |
| 19 | 25 | 8 | 87,5 | |
| 23 | 5 | 8 | 12,5 | |
| | | | | |

| Vergleichsverbindungen: | | | | |
|---|---|---|---|---|
| Flunarizin | 50 | 8 | 100 | |
| Nimodipin | 20 | 8 | 25 | 25,4 |
| Verapamil | 50 | 8 | 50 | |
| Dextromethorphan | 100 | 8 | 100 | 58 |
| | | | | |
| Diazepam | 5 | 8 | 25 | |
| Valproinsäure | 250 | 8 | 87,5 | |
| Carbamazepin | 50 | 8 | 75 | |
| Diphenylhydantoin | 50 | 8 | 50 | |
| Phenobarbital | 50 | 8 | 62,5 | |

**Tabelle 3**

| Passive Pfotenanaphylaxie bei der Ratte | | | |
|---|---|---|---|
| Substanz gemäß Beisp.Nr. | Dosis (mg/kg) p.o. 1 Stunde vor dem Rest | Schutzwirkung (in %) | ED-50 (mg/kg p.o.) |
| 4 | 25 | 79 | |
| 8 | 25 | 35 | |
| 10 | 25 | 22 | |
| 12 | 25 | 12 | |
| 14 | 25 | 81 1) | 2,4 |
| 15 | 25 | 60 | |
| 16 | 25 | 60 | |
| 17 | 25 | 83 | |
| 18 | 25 | 56 | |
| 19 | 25 | 32 | |
| 25 | 25 | 43 | |
| 26 | 25 | 66 | |
| 27 | 25 | 40 | |
| 28 | 25 | 72 | |
| 39 | 25 | 26 | |
| 40 | 25 | 27 | |
| 42 | 25 | 39 | |
| 43 | 25 | 19 | |
| 45 | 25 | 69 | |
| 46 | 25 | 35 | |
| 48 | 25 | 35 | |
| 49 | 25 | 47 | |
| 52 | 25 | 74 | |
| | | | |

| Vergleichsverbindungen: | | | |
|---|---|---|---|
| | | | |
| Astemizol | 10 | 70 | 3,1 |
| Cromoglykat 2) | 2,25 | 58 | 1,1 |
| Diphenhydramin | 100 | 54 | 73 |
| Ketotifen | 30 | 77 | 5,6 |
| Oxatomid | 30 | 75 | 7,3 |
| Picumast | 25 | -21 | - |
| Theophyllin | 60 | 58 | 41,2 |

| | | | |
|---|---|---|---|
| 1) Wirksamkeit bleibt über einen Zeitraum von 24 Stunden voll erhalten | | | |
| 2) Intravenöse Behandlung erfolgte unmittelbar vor dem Test, oral nicht wirksam | | | |

Beispiele für die Herstellung pharmazeutischer Zubereitungen der erfindungsgemäßen Substanzen:
A. Tabletten:
Zur Herstellung von Tabletten, die je nach gewünschter Wirkungsstärke 5 bis 250 mg Wirkstoff enthalten, benötigt man

| | |
|---|---|
| erfindungsgemäße Substanz (Wirkstoff) | 200 bis 5 000 g |
| Zellulosepulver | 2 000 g |
| Maisstärke | 1 200 g |
| kolloide Kieselsäure | 80 g |
| Magnesiumstearat | 20 g |
| Milchzucker | ad 10 000 g |

Der Wirkstoff wird gegebenenfalls gemahlen, mit den Hilfsstoffen homogen vermischt und in der üblichen Weise zu Tabletten von je 250 mg Gewicht und 9 mm Durchmesser verpreßt. Bei Dosierungen über 125 mg preßt man Tabletten von je 500 mg Gewicht und 11 mm Durchmesser. Falls gewünscht, werden die Tabletten mit einem Filmüberzug versehen.
B. Kapseln:
Zur Herstellung von Kapseln, die je nach gewünschter Wirkungsstärke 5 bis 250 mg Wirkstoff enthalten, benötigt man

| | |
|---|---|
| erfindungsgemäße Substanz (Wirkstoff) | 500 bis 12 500 g |
| Maisstärke | 2 000 g |
| kolloide Kieselsäure | 300 g |
| Magnesiumstearat | 50 g |
| Zellulosepulver | ad 20 000 g |

Die feingepulverten Stoffe werden homogen gemischt und in Hartgelatinekapseln der Größe 2 in der Menge von 200 mg pro Kapsel, oder bei Dosierungen über 125 mg in Hartgelatinekapseln der Größe 0 in der Menge von 400 mg pro Kapsel abgefüllt.

## Patentansprüche (Patentansprüche für folgende(n) Vertragsstaat(en): AT, BE, CH, DE, FR, GB, IT, LI, NL)

1. 2H-1-Benzopyran-2-one der allgemeinen Formel I, 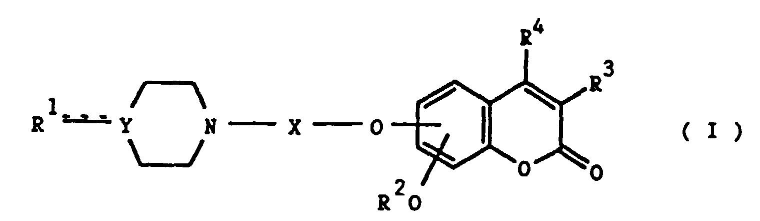 worin
X eine Alkylengruppe mit 2 bis 5 C-Atomen oder eine 2-Hydroxypropylengruppe,
Y ein Stickstoffatom, eine CH-Gruppe, eine COH-Gruppe oder ein Kohlenstoffatom,
R¹ einen Phenyl-, Benzhydryl-, Benzhydryliden-, Benzyl-, Diphenylhydroxymethyl-, Pyridinyl- oder Pyrimidinyl-Rest, der gegebenenfalls mit jeweils ein oder zwei C₁-C₅-Alkylgruppen, mit jeweils ein oder zwei Halogenatomen, mit Halogen und gleichzeitig C₁-C₅-Alkyl, mit Perfluoralkyl mit 1 bis 3 C-Atomen, C₁-C₅-Alkoxy, Methylendioxy, Hydroxy oder Nitro substituiert ist,
R² einen geradkettigen oder verzweigten Alkylrest mit 1 bis 5 C-Atomen oder einen Cycloalkylrest mit 4 bis 6 C-Atomen,
R³ ein Wasserstoffatom, eine Alkylgruppe mit 1 bis 4 C-Atomen oder Phenyl,
R⁴ ein Wasserstoffatom, eine Alkylgruppe mit 1 bis 4 C-Atomen, einen Phenylrest oder einen Trifluormethylrest oder
R³ und R⁴ gemeinsam eine Polymethylenkette -(CH₂)ₙ- mit n = 3 oder 4
darstellen, sowie deren Additionsverbindungen mit physiologisch verträglichen Säuren.

2. Verbindungen der Formel I nach Anspruch 1, worin
X eine Alkylengruppe mit 2 bis 5 C-Atomen,
Y N oder CH,
R¹ einen ggf. mit einem Halogenatom, mit Methyl, Methoxy, Ethoxy oder Hydroxy substituierten Phenyl- oder Diphenylhydroxymethylrest,
R² Methyl oder Ethyl,
R³ ein Wasserstoffatom oder eine Methyl- oder Phenylgruppe,
R⁴ ein Wasserstoffatom, Methyl, Propyl, Isopropyl oder
R³ und R⁴ gemeinsam eine Polymethylenkette -(CH₂)ₙ- mit n = 3 oder 4
bedeuten, sowie deren Additionsverbindungen mit physiologisch verträglichen Säuren.

3. Verfahren zur Herstellung der Verbindungen gemäß Anspruch 1, dadurch gekennzeichnet, daß entweder
a) eine Verbindung der allgemeinen Formel III, 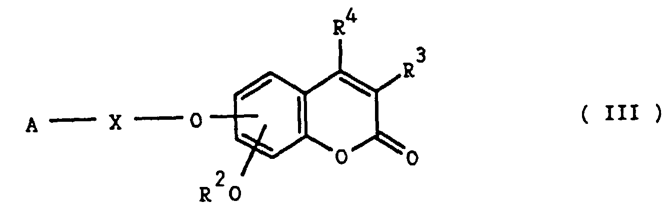 worin X, R², R³ und R⁴ die in Anspruch 1 genannten Bedeutungen haben und A eine Abgangsgruppe ist, die ausgewählt ist aus der Gruppe Chlor, Brom, Iod, Alkylsulfonyloxy, Trifluormethylsulfonyloxy, gegebenenfalls mit Alkyl, Nitro oder Halogen substituiertes Phenylsulfonyloxy, umgesetzt wird mit einer Verbindung der allgemeinen Formel V, 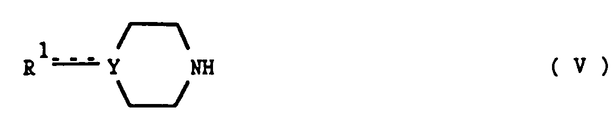 worin R¹ und Y die in Anspruch 1 genannten Bedeutungen haben, oder
b) eine Verbindung der allgemeinen Formel VI, 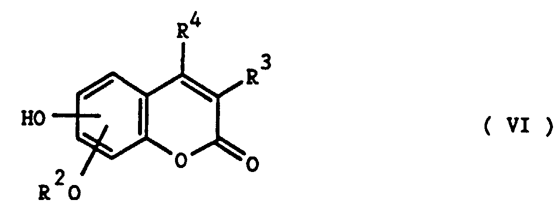 worin R², R³ und R⁴ die in Anspruch 1 genannten Bedeutungen haben,
umgesetzt wird mit einer Verbindung der allgemeinen Formel VII, 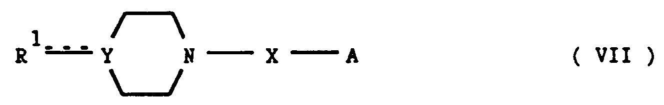 worin X, Y und R¹ die in Anspruch 1 genannten Bedeutungen haben und A die vorstehend unter a) genannten Bedeutungen besitzt,
wonach die gemäß a) oder b) erhaltenen Verbindungen ggf. in ihre physiologisch verträglichen Säureadditionsverbindungen übergeführt werden.

4. Verfahren nach Anspruch 3, dadurch gekennzeichnet, daß die Umsetzung jeweils in Gegenwart einer Base durchgeführt wird, die ausgewählt ist aus der Gruppe der Alkali- und Erdalkalicarbonate, -hydrogencarbonate, -hydride, -alkoholate, -hydroxide und tertiären Amine.

5. Verfahren zur Herstellung derjenigen Verbindungen gemäß Anspruch 1, bei denen die Gruppe X in der allgemeinen Formel I eine 2-Hydroxypropylgruppe ist, dadurch gekennzeichnet, daß eine Verbindung der allgemeinen Formel IV, 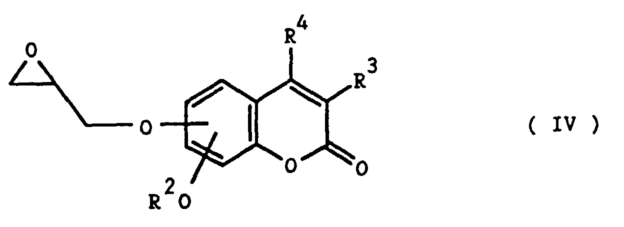 worin R², R³ und R⁴ die in Anspruch 1 angegebenen Bedeutungen haben,
umgesetzt wird mit einer Verbindung der allgemeinen Formel V, 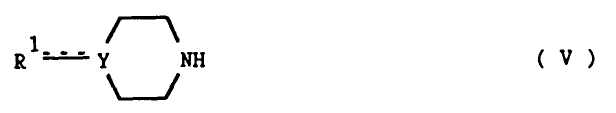 worin R¹ und Y die in Anspruch 1 genannten Bedeutungen besitzen.

6. Verfahren nach einem der Ansprüche 3 bis 5, dadurch gekennzeichnet, daß die Umsetzung in Gegenwart eines gegenüber den Reaktanden inerten Lösungsmittels aus der Gruppe der Alkanole, Methylbenzole, aliphatischen und cycloaliphatischen Ether, Carbonsäuredialkylamide, Tetraalkylharnstoffe, Ketone und Sulfoxide durchgeführt wird.

7. Verfahren nach einem der Ansprüche 3 bis 6, dadurch gekennzeichnet, daß die Umsetzung in Gegenwart eines Katalysators aus der Gruppe der Alkali- und Erdalkaliiodide durchgeführt wird.

8. Verfahren nach einem der Ansprüche 3 bis 7, dadurch gekennzeichnet, daß die Umsetzung bei einer Temperatur zwischen Raumtemperatur und 130°C, vorzugsweise bei einer Temperatur von weniger als 100°C, und bei einem Druck zwischen Normaldruck und 10⁷ Pa durchgeführt wird.

9. Verfahren nach einem der Ansprüche 3 bis 8, dadurch gekennzeichnet, daß die Umsetzung unter einer Schutzgasatmosphäre wie N₂ oder Ar durchgeführt wird.

10. Reaktive Zwischenprodukte der allgemeinen Formel XI, 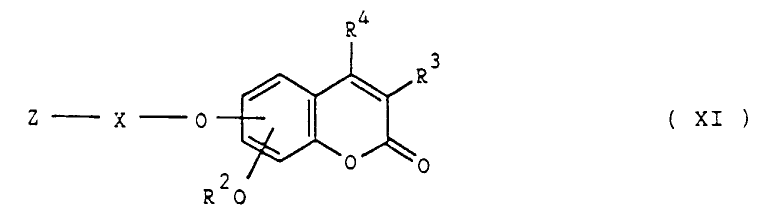 worin R², R³ und R⁴ die in Anspruch 1 angegebenen Bedeutungen haben, und
X eine Alkylengruppe mit 2 bis 5 C-Atomen und
Z eine Hydroxyl- oder Methansulfonyloxygruppe, ein Halogenatom oder
Z-X gemeinsam eine Oxiranylmethylengruppe
bedeuten, mit Ausnahme von 7-Methoxy-6-(oxiranylmethoxy)-2H-1-benzopyran-2-on und 7-Methoxy-4-methyl-6-(oxiranylmethoxy)-2H-1-benzopyran-2-on.

11. Verfahren zur Herstellung derjenigen Verbindungen gemäß Anspruch 10, bei denen die Gruppe X in der allgemeinen Formel XI eine Alkylengruppe und Z eine Hydroxyl- oder Methansulfonyloxygruppe ist, dadurch gekennzeichnet, daß eine Verbindung der allgemeinen Formel VI, 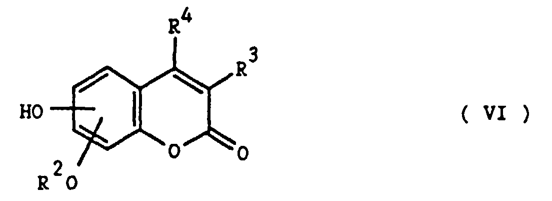 worin R², R³ und R⁴ die in Anspruch 1 genannten Bedeutungen haben, mit einem entsprechenden Halogenalkohol (Hal-X-OH) umgesetzt und anschließend ggf. mit Methansulfonsäurechlorid weiter umgesetzt wird.

12. Verfahren zur Herstellung derjenigen Verbindungen gemäß Anspruch 10, bei denen die Gruppe Z-X- in der allgemeinen Formel XI gemeinsam die Oxiranylmethylengruppe ist, dadurch gekennzeichnet, daß eine Verbindung der allgemeinen Formel VI, 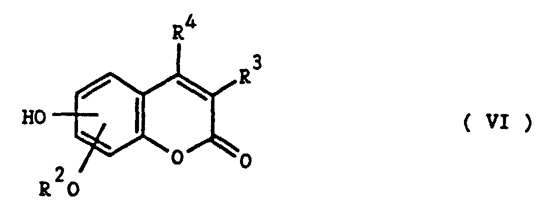 worin R², R³ und R⁴ die in Anspruch 1 genannten Bedeutungen haben, mit Epichlorhydrin oder Epibromhydrin umgesetzt wird.

13. Verfahren zur Herstellung derjenigen Verbindungen gemäß Anspruch 10, die der allgemeinen Formel IX entsprechen, 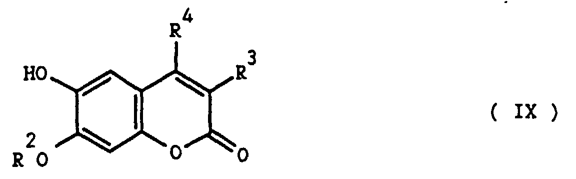 worin R², R³ und R⁴ die in Anspruch 1 angegebenen Bedeutungen haben, jedoch R⁴ nicht Wasserstoff bedeutet, dadurch gekennzeichnet, daß ein 2-Alkoxyhydrochinon der Formel 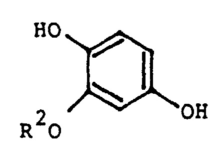 mit einem β-Ketocarbonsäureester der allgemeinen Formel X, 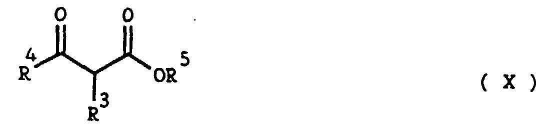 worin R³ und R⁴ die genannten Bedeutungen haben und R⁵ ein Niederalkylrest ist,
in Gegenwart eines sauren Katalysators umgesetzt wird.

14. Verfahren nach Anspruch 13, dadurch gekennzeichnet, daß eine Mineral- oder Lewissäure als Katalysator und gleichzeitig als Lösungsmittel verwendet wird und die Umsetzung bei einer Temperatur zwischen 0 und 60°C durchgeführt wird.

15. Pharmazeutische Zusammensetzung, enthaltend mindestens eine Verbindung gemäß einem der Ansprüche 1 oder 2, ggf. zusammen mit üblichen Hilfs- und Zusatzstoffen.

16. Verwendung der Verbindungen gemäß einem der Ansprüche 1 oder 2 zur Herstellung eines Arzneimittels mit neuroprotektiver, antikonvulsiver und/oder antiepileptischer Wirksamkeit.

17. Verwendung der Verbindungen gemäß einem der Ansprüche 1 oder 2 zur Herstellung eines Arzneimittels mit antiallergischer und antiinflammatorischer Wirksamkeit.

## Patentansprüche (Patentansprüche für folgende(n) Vertragsstaat(en): ES)

1. Verfahren zur Herstellung von 2H-1-Benzopyran-2-onen der allgemeinen Formel I, 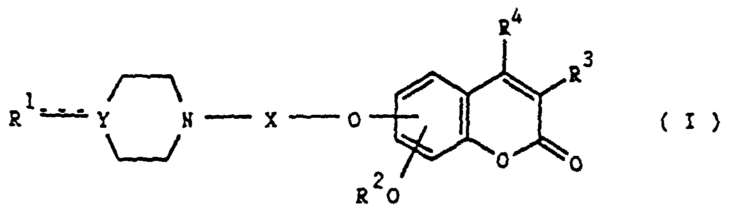 worin
X eine Alkylengruppe mit 2 bis 5 C-Atomen oder eine 2-Hydroxypropylengruppe,
Y ein Stickstoffatom, eine CH-Gruppe, eine COH-Gruppe oder ein Kohlenstoffatom,
R¹ einen Phenyl-, Benzhydryl-, Benzhydryliden-, Benzyl-, Diphenylhydroxymethyl-, Pyridinyl- oder Pyrimidinyl-Rest, der gegebenenfalls mit jeweils ein oder zwei C₁-C₅-Alkylgruppen, mit jeweils ein oder zwei Halogenatomen, mit Halogen und gleichzeitig C₁-C₅-Alkyl, mit Perfluoralkyl mit 1 bis 3 C-Atomen, C₁-C₅-Alkoxy, Methylendioxy, Hydroxy oder Nitro substituiert ist,
R² einen geradkettigen oder verzweigten Alkylrest mit 1 bis 5 C-Atomen oder einen Cycloalkylrest mit 4 bis 6 C-Atomen
R³ ein Wasserstoffatom, eine Alkylgruppe mit 1 bis 4 C-Atomen oder Phenyl,
R⁴ ein Wasserstoffatom, eine Alkylgruppe mit 1 bis 4 C-Atomen, einen Phenylrest oder einen Trifluormethylrest oder
R³ und R⁴ gemeinsam eine Polymethylenkette -(CH₂)ₙ- mit n = 3 oder 4
darstellen, sowie von deren Additionsverbindungen mit physiologisch verträglichen Säuren, dadurch gekennzeichnet, daß entweder
a) eine Verbindung der allgemeinen Formel III, 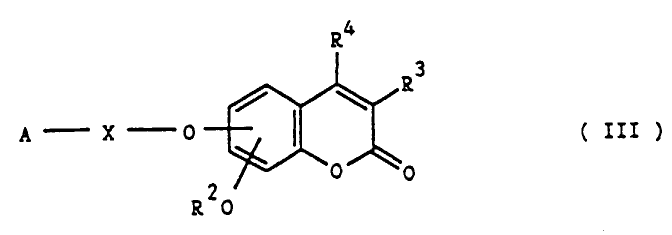 worin X, R², R³ und R⁴ die oben genannten Bedeutungen haben und A eine Abgangsgruppe ist, die ausgewählt ist aus der Gruppe Chlor, Brom, Iod, Alkylsulfonyloxy, Trifluormethylsulfonyloxy, gegebenenfalls mit Alkyl, Nitro oder Halogen substituiertes Phenylsulfonyloxy, umgesetzt wird mit einer Verbindung der allgemeinen Formel V, 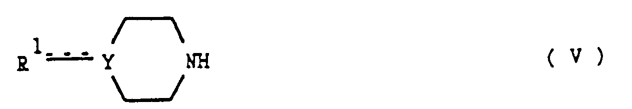 worin R¹ und Y die oben genannten Bedeutungen haben, oder
b) eine Verbindung der allgemeinen Formel VI, 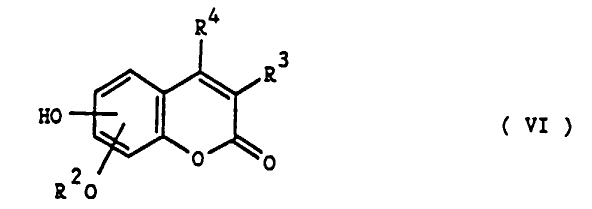 worin R², R³ und R⁴ die oben genannten Bedeutungen haben,
umgesetzt wird mit einer Verbindung der allgemeinen Formel VII, 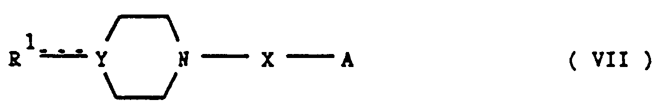 worin X, Y und R¹ die oben genannten Bedeutungen haben und A die vorstehend unter a) genannten Bedeutungen besitzt,
wonach die gemäß a) oder b) erhaltenen Verbindungen ggf. in ihre physiologisch verträglichen Säureadditionsverbindungen übergeführt werden.

2. Verfahren nach Anspruch 1, dadurch gekennzeichnet, daß die Umsetzung jeweils in Gegenwart einer Base durchgeführt wird, die ausgewählt ist aus der Gruppe der Alkali- und Erdalkalicarbonate, -hydrogencarbonate, -hydride, -alkoholate, -hydroxide und tertiären Amine.

3. Verfahren zur Herstellung derjenigen Verbindungen der allgemeinen Formel I, bei denen die Gruppe X eine 2-Hydroxypropylgruppe ist, dadurch gekennzeichnet, daß eine Verbindung der allgemeinen Formel IV, 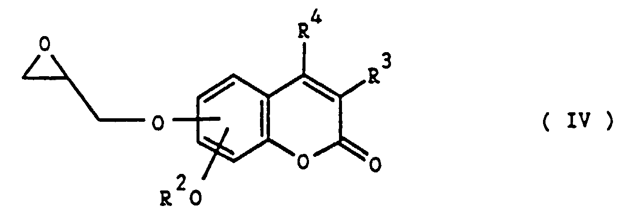 worin R², R³ und R⁴ die in Anspruch 1 angegebenen Bedeutungen haben,
umgesetzt wird mit einer Verbindung der allgemeinen Formel V, 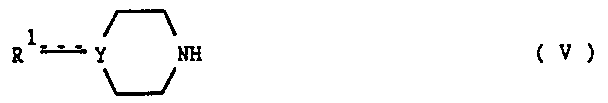 worin R¹ und Y die in Anspruch 1 genannten Bedeutungen besitzen.

4. Verfahren nach einem der Ansprüche 1 bis 3, dadurch gekennzeichnet, daß die Umsetzung in Gegenwart eines gegenüber den Reaktanden inerten Lösungsmittels aus der Gruppe der Alkanole, Methylbenzole, aliphatischen und cycloaliphatischen Ether, Carbonsäuredialkylamide, Tetraalkylharnstoffe, Ketone und Sulfoxide durchgeführt wird.

5. Verfahren nach einem der Ansprüche 1 bis 4, dadurch gekennzeichnet, daß die Umsetzung in Gegenwart eines Katalysators aus der Gruppe der Alkali- und Erdalkaliiodide durchgeführt wird.

6. Verfahren nach einem der Ansprüche 1 bis 5, dadurch gekennzeichnet, daß die Umsetzung bei einer Temperatur zwischen Raumtemperatur und 130°C, vorzugsweise bei einer Temperatur von weniger als 100°C, und bei einem Druck zwischen Normaldruck und 10⁷ Pa durchgeführt wird.

7. Verfahren nach einem der Ansprüche 1 bis 6, dadurch gekennzeichnet, daß die Umsetzung unter einer Schutzgasatmosphäre wie N₂ oder Ar durchgeführt wird.

8. Verfahren zur Herstellung von reaktiven Zwischenprodukten der allgemeinen Formel XI, 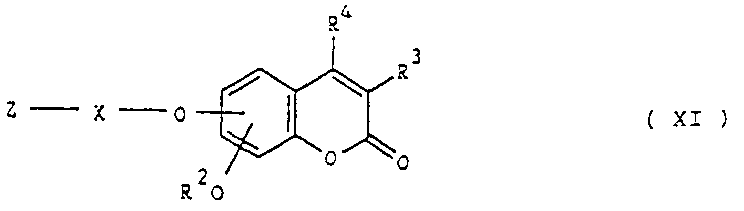 worin R², R³ und R⁴ die in Anspruch 1 angegebenen Bedeutungen haben, und
X eine Alkylengruppe mit 2 bis 5 C-Atomen und
Z eine Hydroxyl- oder Methansulfonyloxygruppe
bedeuten, dadurch gekennzeichnet, daß eine Verbindung der allgemeinen Formel VI, 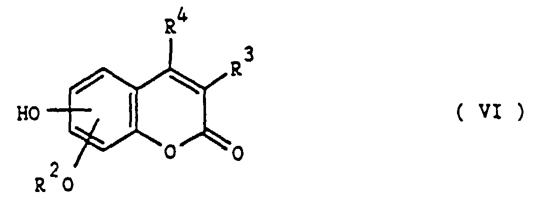 worin R², R³ und R⁴ die in Anspruch 1 genannten Bedeutungen haben, mit einem entsprechenden Halogenalkohol (Hal-X-OH) Umgesetzt und anschließend ggf. mit Methansulfonsäurechlorid weiter umgesetzt wird.

9. Verfahren zur Herstellung von reaktiven Zwischenprodukten der allgemeinen Formel XI, 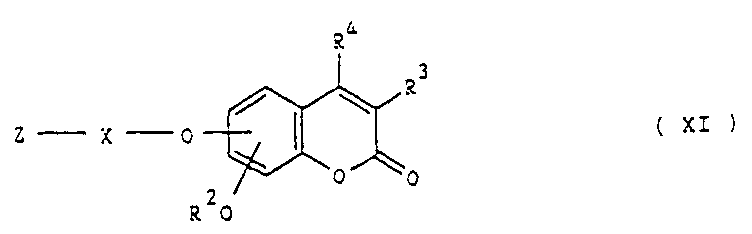 worin R², R³ und R⁴ die in Anspuch 1 angegebenen Bedeutungen haben, und
Z-X gemeinsam eine Oxiranylmethylengruppe bedeuten, mit Ausnahme von 7-Methoxy-6-(oxiranylmethoxy)-2H-1-benzopyran-2-on und 7-Methoxy-4-methyl-6-(oxiranylmethoxy)-2H-1-benzopyran-2-on, dadurch gekennzeichnet, daß eine Verbindung der allgemeinen Formel VI, 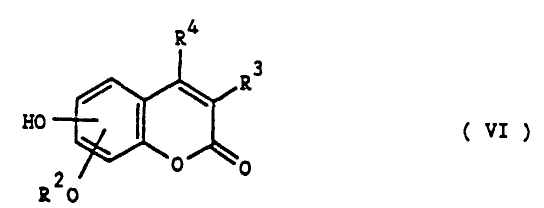 worin R², R³ und R⁴ die in Anspruch 1 genannten Bedeutungen haben, mit Epichlorhydrin oder Epibromhydrin umgesetzt wird.

10. Verfahren zur Herstellung von reaktiven Zwischenprodukten der allgemeinen Formel IX, 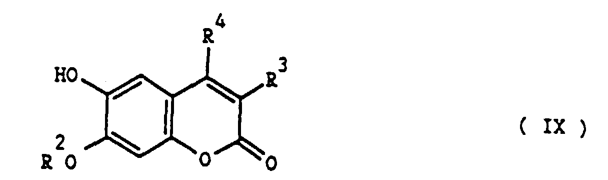 worin R², R³ und R⁴ die in Anspruch 1 angegebenen Bedeutungen haben, jedoch R⁴ nicht Wasserstoff bedeutet, dadurch gekennzeichnet, daß ein 2-Alkoxyhydrochinon der Formel 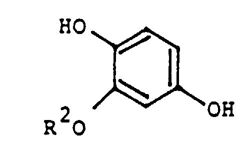 mit einem β-Ketocarbonsäureester der allgemeinen Formel X, 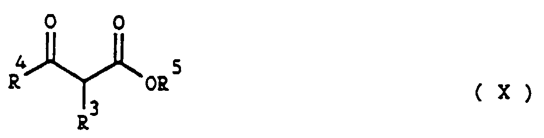 worin R³ und R⁴ die genannten Bedeutungen haben und R⁵ ein Niederalkylrest ist,
in Gegenwart eines sauren Katalysators umgesetzt wird.

11. Verfahren nach Anspruch 10, dadurch gekennzeichnet, daß eine Mineral- oder Lewissäure als Katalysator und gleichzeitig als Lösungsmittel verwendet wird und die Umsetzung bei einer Temperatur zwischen 0 und 60°C durchgeführt wird.

## Claims (Claims for the following Contracting State(s): AT, BE, CH, DE, FR, GB, IT, LI, NL)

1. 2H-1-benzopyran-2-ones of the general formula I, 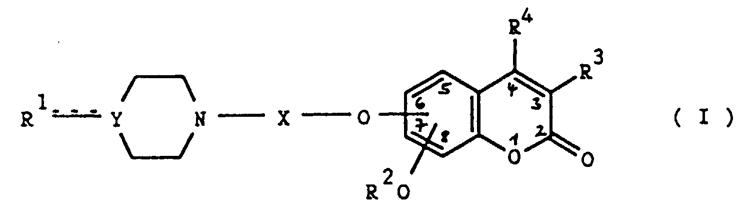 wherein:
X denotes an alkylene group with 2 to 5 C atoms or a 2-hydroxypropylene group,
Y denotes a nitrogen atom, a CH- group, a COH- group or a carbon atom,
R¹ denotes a phenyl, benzhydryl, benzhydrylidene, benzyl, diphenylhydroxymethyl, pyridinyl or pyrimidinyl radical, which is optionally substituted with respectively one or two C₁-C₅ alkyl groups, with respectively one or two halogen atoms, with halogen and simultaneously C₁-C₅ alkyl, with perfluoroalkyl with 1 to 3 C atoms, C₁-C₅ alkoxy, methylenedioxy, hydroxy or nitro,
R² denotes a straight chained or branched alkyl radical with 1 to 5 C atoms or a cycloalkyl radical with 4 to 6 C atoms,
R³ denotes a hydrogen atom, an alkyl group with 1 to 4 C atoms or a phenyl radical,
R⁴ denotes a hydrogen atom, an alkyl group with 1 to 4 C atoms, a phenyl radical or a trifluoromethyl radical or
R³ and R⁴ jointly denote a polymethylene chain -(CH₂)ₙ- with n equal to 3 or 4,
and the addition compounds thereof with physiologically compatible acids.

2. The compounds of formula I as claimed in claim 1 wherein
X denotes an alkylene group with 2 to 5 C atoms,
Y denotes N or CH,
R¹ denotes a phenyl or diphenylhydroxymethyl radical with optional substitution by a halogen atom, methyl, methoxy, ethoxy or hydroxy,
R² denotes methyl or ethyl,
R³ denotes a hydrogen atom or a methyl or phenyl group,
R⁴ denotes a hydrogen atom, methyl, propyl, isopropyl or
R³ and R⁴ jointly denote a polymethylene chain -(CH₂)ₙ- in which n is equal to 3 or 4,
and the addition compounds thereof with physiologically compatible acids.

3. A method for the production of the compounds as claimed in claim 1, characterized in that either
a) a compound of the general formula III 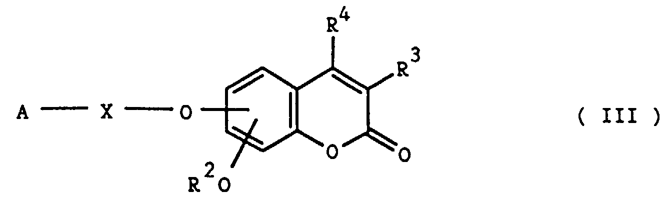 wherein X, R², R³ and R⁴ have the meanings as given in claim 1 and A denotes a leaving group selected from the group consisting of chlorine, bromine, iodine, alkylsulfonyloxy, trifluoromethylsulfonyloxy and phenylsulfonyloxy which is optionally substituted with alkyl, nitro or halogen, is reacted with a compound of the general formula V 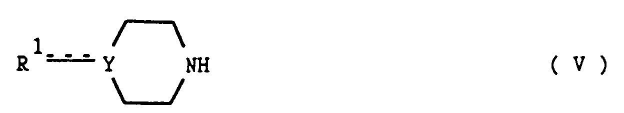 wherein R¹ and Y have the meanings given in claim 1, or
b) a compound of the general formula VI, 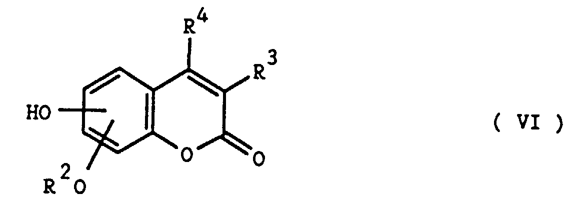 wherein R², R³ and R⁴ have the meanings given in claim 1 is reacted with a compound of the general formula VII, 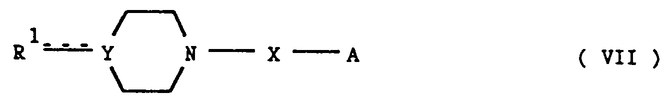 wherein X, Y and R¹ have the meanings given in claim 1 and A has the meanings as above noted in (a),
whereafter both in the case of (a) and also in the case of (b) the compounds obtained are optionally converted into their physiologically compatible acid addition compounds.

4. The method as claimed in claim 3, characterized in that reaction respectively takes place in the presence of a base selected from the group consisting of alkali metal or alkaline earth metal carbonates, hydrogencarbonates, hydrides, alcoholates, hydroxides, and tertiary amines.

5. A method for the production of those compounds of claim 1 in the case of which the group X in the general formula I is a 2-hydroxypropylene group, characterized in that a compound of the general formula IV 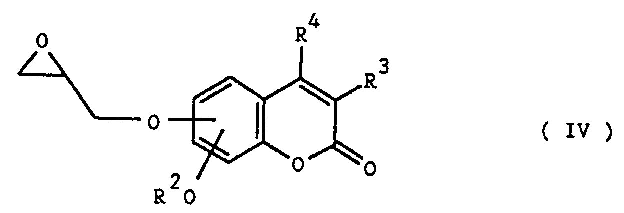 wherein R², R³ and R⁴ have the meanings given in claim 1, is reacted with a compound of the general formula V, 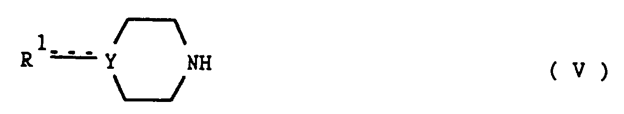 wherein R¹ and Y have the meanings given in claim 1.

6. The method of any one of claims 3 through 5, characterized in that the reaction is performed in the presence of a solvent which is inert with respect to the reactants and is selected from the group consisting of alkanols, methylbenzenes, aliphatic or cycloaliphatic ethers, carboxylic acid dialkylamides, tetraalkylureas, ketones, and sulfoxides.

7. The method of any one of claims 3 through 6, characterized in that the reaction is performed in the presence of a catalyst selected from the group consisting of alkali metal iodides and alkaline earth metal iodides.

8. The method of any one of claims 3 through 7, characterized in that the reaction is performed at a temperature which is between room temperature and 130° C, preferably at a temperature of less than 100° C, and at a pressure between normal pressure and 10⁷ Pa.

9. The method of any one of claims 3 through 8, characterized in that the reaction is performed in a protective gas atmosphere such as N₂ or Ar.

10. Reactive intermediates of the general formula XI, 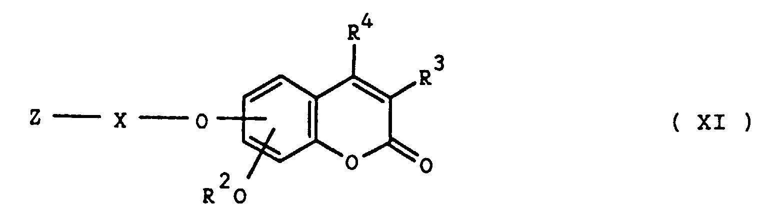 wherein R², R³ and R⁴ have the meanings given in claim 1, and
X denotes an alkylene group with 2 to 5 C atoms and
Z denotes a hydroxyl group or a methanesulfonyloxy group, a halogen atom or
Z-X jointly denote an oxiranylmethylene group, with the exclusion, however, of 7-methoxy-6-(oxiranylmethoxy)-2H-l-benzopyran-2-one and 7-methoxy-4-methyl-6-(oxiranylmethoxy)-2H-1-benzopyran-2-one.

11. A method for the production of those compounds of claim 10 in the case of which the group X in the general formula XI is an alkylene group and Z is hydroxyl or methanesulfonyloxy, characterized in that a compound of the general formula VI, 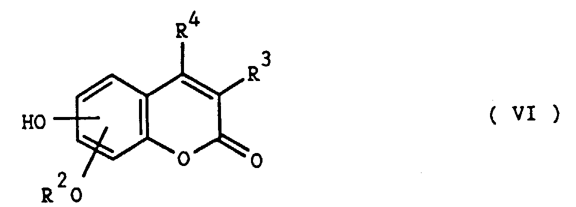 wherein R², R³ and R⁴ have the meanings given in claim 1 is reacted with a corresponding halogen alcohol (Hal-X-OH) and thereafter optionally further reacted with methanesulfonic acid chloride.

12. A method for the production of those compounds of claim 10 in the case of which the group Z-X in the general formula XI is jointly the oxiranylmethylene group, characterized in that a compound of the general formula VI, 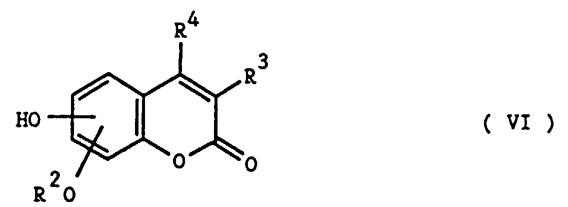 wherein R², R³ and R⁴ have the meanings given in claim 1 is reacted with epichlorohydrin or epibromohydrin.

13. A method for the production of those compounds of claim 10 which comply with the general formula IX, 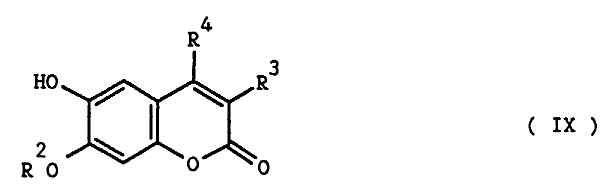 wherein R², R³ and R⁴ have the meanings given in claim 1 but R⁴ does not have the meaning of hydrogen, characterized in that a 2-alkoxyhydroquinone of the formula 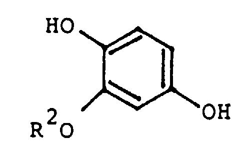 is reacted with a β-ketocarboxylic acid ester of the general formula X 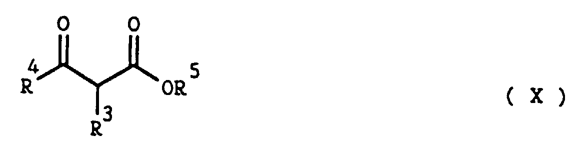 wherein R³ and R⁴ have the meanings given and R⁵ is a lower alkyl radical, in the presence of an acidic catalyst.

14. The method of claim 13, characterized in that a mineral or Lewis acid is utilized as a catalyst and simultaneously as a solvent and in that the reaction is carried out at a temperature between 0° C and 60° C.

15. A pharmaceutical preparation containing at least one compound as claimed in claim 1 or in claim 2 optionally together with conventional adjuvants and additives.

16. The use of the compounds as claimed in claim 1 or in claim 2 for the production of a medicament with a neuroprotective, anticonvulsive and/or antiepileptic action.

17. The use of the compounds as claimed in claim 1 or in claim 2 for the production of a medicament with an anti-allergic and anti-inflammatory action.

## Claims (Claims for the following Contracting State(s): ES)

1. A method for the production of 2H-1-benzopyran-2-ones of the general formula I, 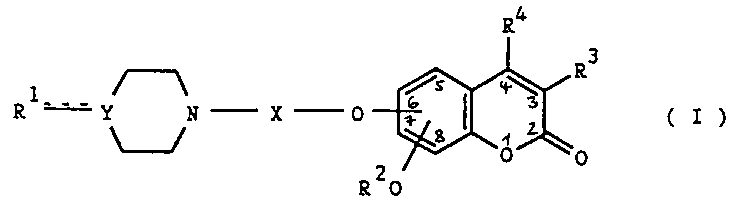 wherein:
X denotes an alkylene group with 2 to 5 C atoms or a 2-hydroxypropylene group,
Y denotes a nitrogen atom, a CH- group, a COH- group or a carbon atom,
R¹ denotes a phenyl, benzhydryl, benzhydrylidene, benzyl, diphenylhydroxymethyl, pyridinyl or pyrimidinyl radical, which is optionally substituted with respectively one or two C₁-C₅ alkyl groups, with respectively one or two halogen atoms, with halogen and simultaneously C₁-C₅ alkyl, with perfluoroalkyl with 1 to 3 C atoms, C₁-C₅ alkoxy, methylenedioxy, hydroxy or nitro,
R² denotes a straight chained or branched alkyl radical with 1 to 5 C atoms or a cycloalkyl radical with 4 to 6 C atoms,
R³ denotes a hydrogen atom, an alkyl group with 1 to 4 C atoms or a phenyl radical,
R⁴ denotes a hydrogen atom, an alkyl group with 1 to 4 C atoms, a phenyl radical or a trifluoromethyl radical or
R³ and R⁴ jointly denote a polymethylene chain -(CH₂)ₙ- with n equal to 3 or 4,
and of the addition compounds thereof with physiologically compatible acids, characterized in that either
a) a compound of the general formula III 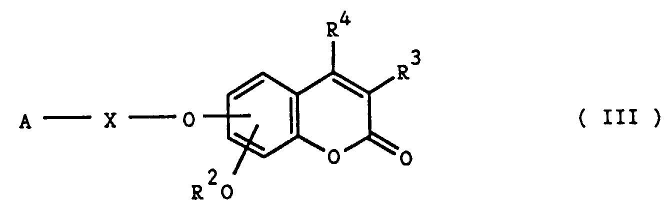 wherein X, R², R³ and R⁴ have the meanings as given above and A denotes a leaving group selected from the group consisting of chlorine, bromine, iodine, alkylsulfonyloxy, trifluoromethylsulfonyloxy and phenylsulfonyloxy which is optionally substituted with alkyl, nitro or halogen, is reacted with a compound of the general formula V 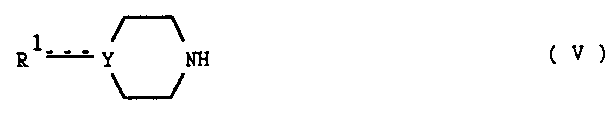 wherein R¹ and Y have the meanings given above, or
b) a compound of the general formula VI, 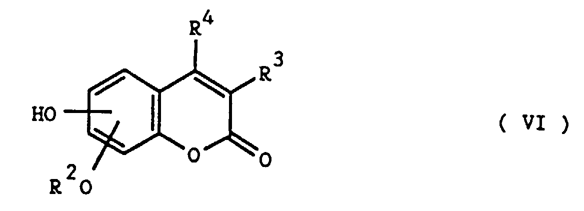 wherein R², R³ and R⁴ have the above noted meanings is reacted with a compound of the general formula VII, 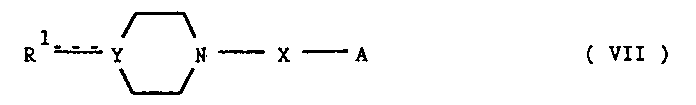 wherein X, Y and R¹ have the meanings given above and A has the meanings as above noted in (a),
whereafter both in the case of (a) and also in the case of (b) the compounds obtained are optionally converted into their physiologically compatible acid addition compounds.

2. The method of claim 1, characterized in that reaction respectively takes place in the presence of a base selected from the group consisting of alkali metal or alkaline earth metal carbonates, hydrogencarbonates, hydrides, alcoholates, hydroxides, and tertiary amines.

3. A method for the production of those compounds of general formula I in the case of which the group X is a 2-hydroxypropylene group, characterized in that a compound of the general formula IV 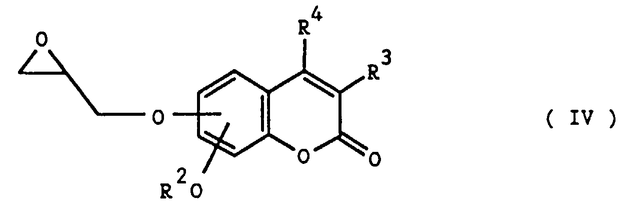 wherein R², R³ and R⁴ have the meanings given in claim 1, is reacted with a compound of the general formula V, 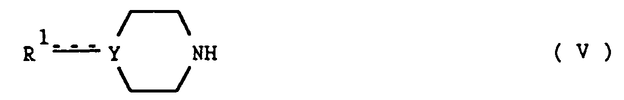 wherein R¹ and Y have the meanings given in claim 1.

4. The method of any one of claims 1 to 3, characterized in that the reaction is performed in the presence of a solvent which is inert with respect to the reactants and is selected from the group consisting of alkanols, methylbenzenes, aliphatic or cycloaliphatic ethers, carboxylic acid dialkylamides, tetraalkylureas, ketones, and sulfoxides.

5. The method of any one of claims 1 to 4, characterized in that the reaction is performed in the presence of a catalyst selected from the group consisting of alkali metal iodides, and alkaline earth metal iodides.

6. The method of any one of claims 1 to 5, characterized in that the reaction is performed at a temperature which is between room temperature and 130° C, preferably at a temperature of less than 100° C, and at a pressure between normal pressure and 10⁷ Pa.

7. The method of any one of claims 1 to 6, characterized in that the reaction is performed in a protective gas atmosphere such as N₂ or Ar.

8. A method for the production of reactive intermediates of the general formula XI, 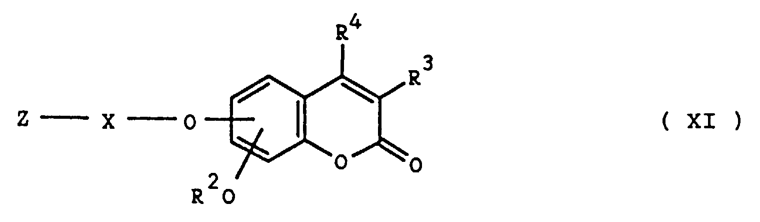 wherein R², R³ and R⁴ have the meanings given in claim 1, and
X denotes an alkylene group with 2 to 5 C atoms and
Z denotes a hydroxyl group or a methanesulfonyloxy group,
characterized in that a compound of the general formula VI, 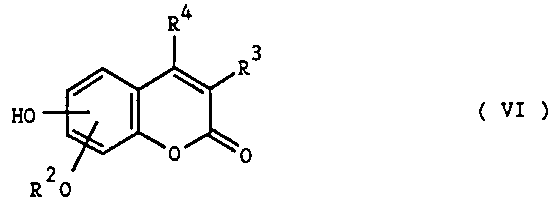 wherein R², R³ and R⁴ have the meanings given in claim 1 is reacted with a corresponding halogen alcohol (Hal-X-OH) and thereafter optionally further reacted with methanesulfonic acid chloride.

9. A method for the production of reactive intermediates of the general formula XI, 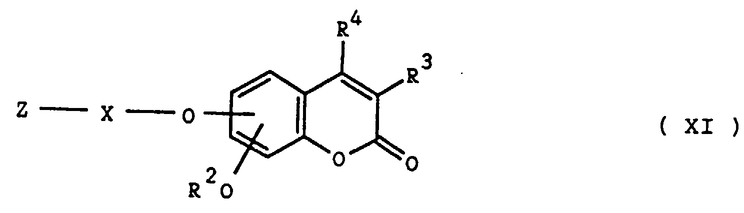 wherein R², R³ and R⁴ have the meanings given in claim 1, and
Z-X jointly denote an oxiranylmethylene group with the exclusion, however, of 7-methoxy-6-(oxiranylmethoxy)-2H-1-benzopyran-2-one and 7-methoxy-4-methyl-6-(oxiranylmethoxy)-2H-1-benzopyran-2-one, characterized in that a compound of the general formula VI, 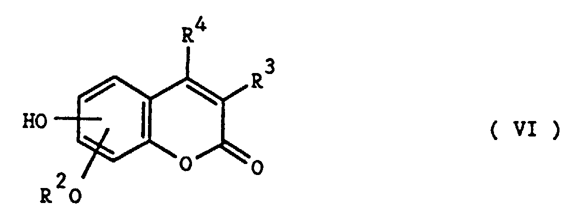 wherein R², R³ and R⁴ have the meanings given in claim 1 is reacted with epichlorohydrin or epibromohydrin.

10. A method for the production of reactive intermediates of the general formula IX, 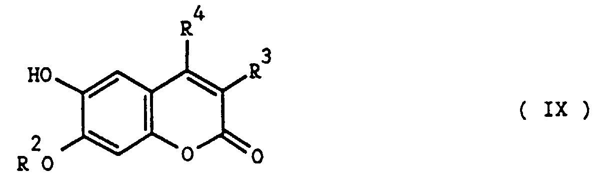 wherein R², R³ and R⁴ have the meanings given in claim 1 but R⁴ does not have the meaning of hydrogen, characterized in that a 2-alkoxyhydroquinone of the formula 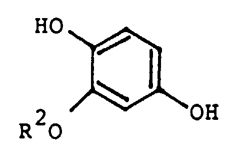 is reacted with a β-ketocarboxylic acid ester of the general formula X 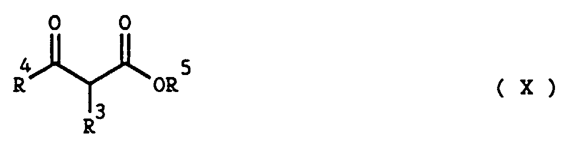 wherein R³ and R⁴ have the meanings given and R⁵ is a lower alkyl radical, in the presence of an acidic catalyst.

11. The method of claim 10, characterized in that a mineral or Lewis acid is utilized as a catalyst and simultaneously as a solvent and in that the reaction is carried out at a temperature between 0° C and 60° C.

## Revendications (Revendications pour l'(les) Etat(s) contractant(s) suivant(s): AT, BE, CH, LI, DE, FR, GB, IT, NL)

1. 2H-1-benzopyranne-2-ones de formule générale I, 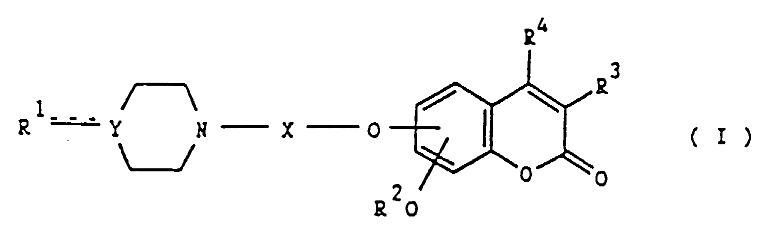 dans laquelle
X est un groupe alkylène ayant de 2 à 5 atomes de carbone ou un groupe 2-hydroxypropylène,
Y est un atome d'azote, un groupe CH, un groupe COH ou un atome de carbone,
R¹ est un résidu phényle, benzhydryle, benzhydrylidène, benzyle, diphénylhydroxyméthyle, pyridinyle ou pyrimidinyle, ce résidu étant éventuellement substitué à chaque fois par un ou deux groupes alkyle en C₁ à C₅, par un ou deux atomes d'halogène, par un halogène et simultanément un groupe alkyle en C₁ à C₅, par un groupe perfluoralkyle ayant de 1 à 3 atomes de carbone, alcoxy en C₁ à C₅, méthylènedioxy, hydroxy ou nitro,
R² est un radical alkyle à chaîne droite ou ramifiée ayant de 1 à 5 atomes de carbone, ou un radical cycloalkyle ayant de 4 à 6 atomes de carbone,
R³ est un atome d'hydrogène, un groupe alkyle ayant de 1 à 4 atomes de carbone, ou le groupe phényle,
R⁴ est un atome d'hydrogène, un groupe alkyle ayant de 1 à 4 atomes de carbone, le radical phényle ou le radical trifluorométhyle, ou bien
R³ et R⁴ représentent ensemble une chaîne polyméthylène -(CH₂)ₙ- avec n = 3 ou 4,
ainsi que leurs composés d'addition avec des acides acceptables d'un point de vue physiologique.

2. Composés de formule I selon la revendication 1, dans laquelle
X est un groupe alkylène ayant de 2 à 5 atomes de carbone,
Y est N ou CH,
R¹ est un radical phényl- ou diphénylhydroxyméthyle éventuellement substitué par un atome d'halogène ou par un substituant méthyle, méthoxy, éthoxy ou hydroxy,
R² est le radical méthyle ou éthyle,
R³ est un atome d'hydrogène ou le groupe méthyle ou phényle,
R⁴ est un atome d'hydrogène ou le groupe méthyle, propyle ou isopropyle, ou bien
R³ et R⁴ représentent ensemble une chaîne polyméthylène -(CH₂)ₙ- avec n = 3 ou 4,
ainsi que leurs composés d'addition avec des acides acceptables d'un point de vue physiologique.

3. Procédé pour préparer les composés selon la revendication 1, caractérisé en ce que, soit
a) on fait réagir un composé de formule générale III, 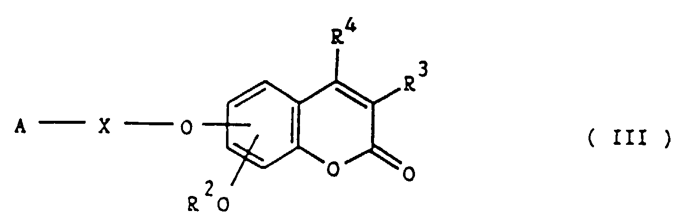 dans laquelle X, R², R³ et R⁴ ont les significations données dans la revendication 1 et où A est un groupe partant, qui est choisi parmi l'ensemble comprenant les radicaux chloro, bromo, iodo, alkylsulfonyloxy, trifluorométhylsulfonyloxy, éventuellement phénylsulfonyloxy substitué par des substituants alkyle, nitro ou halogéno, avec un composé de formule générale V, 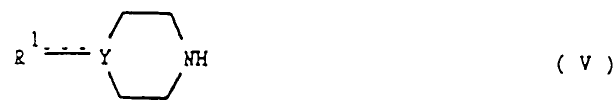 dans laquelle R¹ et Y ont les significations données dans la revendication 1, ou bien
b) on fait réagir un composé de formule générale VI, 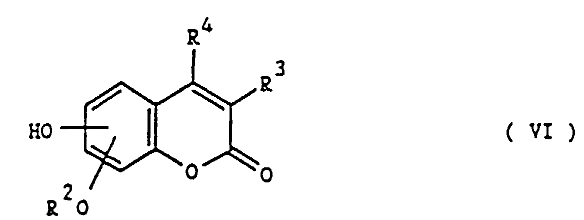 dans laquelle R², R³ et R⁴ ont les significations données dans la revendication 1, avec un composé de formule générale VII, 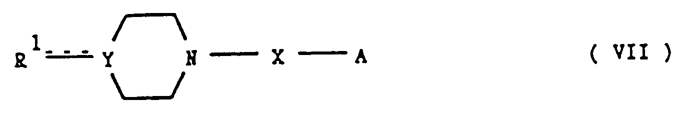 dans laquelle X, Y et R¹ ont les significations données dans la revendication 1, et A a les significations données ci-dessus en a),
ce après quoi les composés obtenus selon a) ou b) sont convertis éventuellement en leurs composés d'addition avec un acide, acceptables d'un point de vue physiologique.

4. Procédé selon la revendication 3, caractérisé en ce que la réaction s'effectue dans tous les cas en présence d'une base, qui est choisie parmi l'ensemble comprenant les carbonates alcalins, les carbonates alcalino-terreux, les hydrogénocarbonates, les hydrures, les alcoolates, les hydroxydes et les amines tertiaires.

5. Procédé pour préparer les composés selon la revendication 1 dans lesquels le groupe X, de formule générale I, est un groupe 2-hydroxypropyle, caractérisé en ce qu'on fait réagir un composé de formule générale IV, 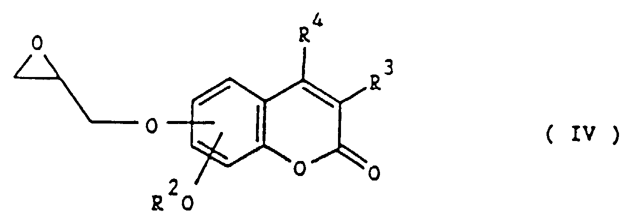 dans laquelle R², R³ et R⁴ ont les significations données dans la revendication 1, avec un composé de formule générale V, 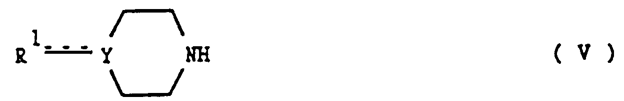 dans laquelle R¹ et Y ont les significations données dans la revendication 1.

6. Procédé selon l'une des revendications 3 à 5, caractérisé en ce que la réaction est mise en oeuvre en présence d'un solvant inerte vis-à-vis des réactants, choisi parmi l'ensemble comprenant les alcanols, les méthylbenzènes, les éthers aliphatiques et cycloaliphatiques, les dialkylamides d'acides carboxyliques, les tétraalkylurées, les cétones et les sulfoxydes.

7. Procédé selon l'une des revendications 3 à 6, caractérisé en ce que la réaction est mise en oeuvre en présence d'un catalyseur choisi parmi l'ensemble comprenant les iodures alcalins et alcalino-terreux.

8. Procédé selon l'une des revendications 3 à 7, caractérisé en ce que la réaction est mise en oeuvre à une température comprise entre la température ambiante et 130°C, de préférence à une température inférieure à 100°C, et sous une pression comprise entre la pression normale et 10⁷ Pa.

9. Procédé selon l'une des revendications 3 à 8, caractérisé en ce que la réaction est mise en oeuvre sous une atmosphère d'un gaz protecteur tel que N₂ ou Ar.

10. Produits intermédiaires réactifs de formule générale XI 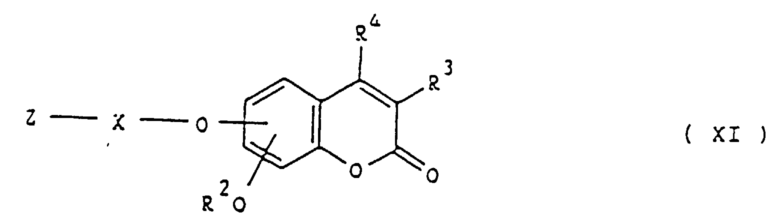 dans laquelle R², R³ et R⁴ ont les significations données dans la revendication 1, et
X est un groupe alkylène ayant de 2 à 5 atomes de carbone, et
Z est un groupe hydroxyle ou méthanesulfonyloxy, un atome d'halogène, ou bien
Z et X représentent ensemble un groupe oxirannylméthylène, à l'exception de la 7-méthoxy-6-(oxirannylméthoxy)-2H-1-benzopyranne-2-one et de la 7-méthoxy-4-méthyl-6-(oxirannylméthoxy)-2H-1-benzopyranne-2-one.

11. Procédé pour préparer les composés selon la revendication 10, dans lesquels le groupe X, dans la formule générale XI, est un groupe alkylène et Z est un groupe hydroxyl- ou méthanesulfonyloxy, caractérisé en ce qu'on fait réagir un composé de formule générale VI 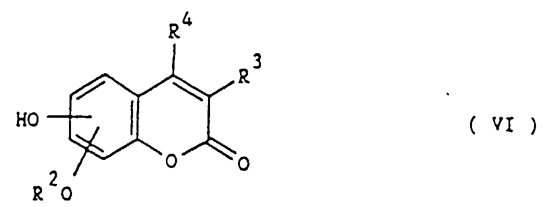 dans laquelle R², R³ et R⁴ ont les significations données dans la revendication 1, avec un halogénoalcool correspondant (Hal-X-OH), puis on le soumet éventuellement à une réaction plus poussée avec le chlorure de méthanesulfonyle.

12. Procédé pour préparer les composés selon la revendication 10 dans lesquels les groupes Z et X de la formule générale XI forment ensemble le groupe oxyrannylméthylène, caractérisé en ce qu'on fait réagir un composé de formule générale VI 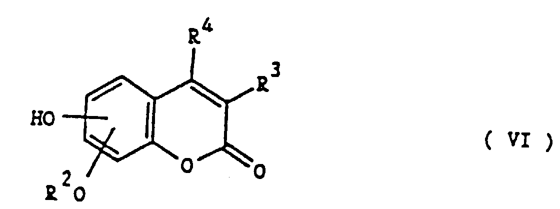 dans laquelle R², R³ et R⁴ ont les significations données dans la revendication 1, avec de l'épichlorhydrine ou de l'épibromhydrine.

13. Procédé pour préparer les composés selon la revendication 10, correspondant à la formule générale IX, 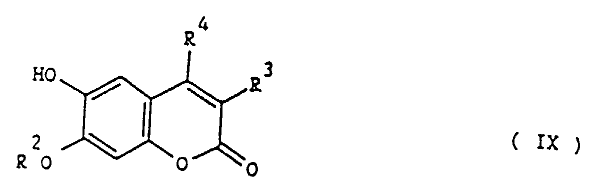 dans laquelle R², R³ et R⁴ ont les significations données dans la revendication 1 mais R⁴ n'est pas un hydrogène, caractérisé en ce qu'on fait réagir une 2-alcoxyhydroquinone de formule 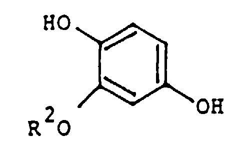 avec un ester d'un acide β-cétocarboxylique de formule générale X, 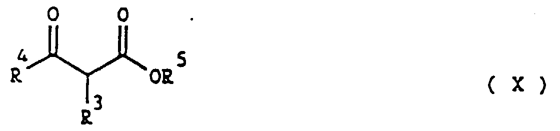 dans laquelle R³ et R⁴ ont les significations données ci-dessus, et R⁵ est un radical alkyle inférieur,
en présence d'un catalyseur acide.

14. Procédé selon la revendication 13, caractérisé en ce qu'on utilise un acide minéral ou un acide de Lewis comme catalyseur et simultanément comme solvant, la réaction étant mise en oeuvre à une température comprise entre 0 et 60°C.

15. Composition pharmaceutique contenant au moins un composé selon l'une des revendications 1 ou 2, éventuellement en même temps que des adjuvants et additifs usuels.

16. Utilisation des composés selon l'une des revendications 1 ou 2, pour préparer un médicament ayant une activité neuroprotectrice, anticonvulsivante et/ou anti-épileptique.

17. Utilisation des composés selon l'une des revendications 1 ou 2, pour préparer un médicament ayant une activité anti-allergique et anti-inflammatoire.

## Revendications (Revendications pour l'(les) Etat(s) contractant(s) suivant(s): ES)

1. Procédé pour préparer des 2H-1-benzopyranne-2-ones de formule générale I, 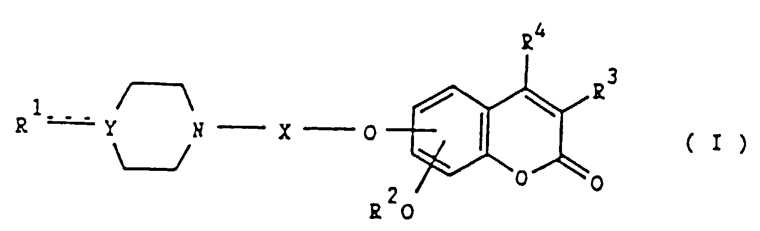 dans laquelle
X est un groupe alkylène ayant de 2 à 5 atomes de carbone ou un groupe 2-hydroxypropylène,
Y est un atome d'azote, un groupe CH, un groupe COH ou un atome de carbone,
R¹ est un résidu phényle, benzhydryle, benzhydrylidène, benzyle, diphénylhydroxyméthyle, pyridinyle ou pyrimidinyle, ce résidu étant éventuellement substitué à chaque fois par un ou deux groupes alkyle en C₁ à C₅, par un ou deux atomes d'halogène, par un halogène et simultanément un groupe alkyle en C₁ à C₅, par un groupe perfluoralkyle ayant de 1 à 3 atomes de carbone, alcoxy en C₁ à C₅, méthylènedioxy, hydroxy ou nitro,
R² est un radical alkyle à chaîne droite ou ramifiée ayant de 1 à 5 atomes de carbone, ou un radical cycloalkyle ayant de 4 à 6 atomes de carbone,
R³ est un atome d'hydrogène, un groupe alkyle ayant de 1 à 4 atomes de carbone, ou le groupe phényle,
R⁴ est un atome d'hydrogène, un groupe alkyle ayant de 1 à 4 atomes de carbone, le radical phényle ou le radical trifluorométhyle, ou bien
R³ et R⁴ représentent ensemble une chaîne polyméthylène -(CH₂)ₙ- avec n = 3 ou 4,
ainsi que leurs composés d'addition avec des acides acceptables d'un point de vue physiologique, caractérisé en ce que, soit
a) on fait réagir un composé de formule générale III, 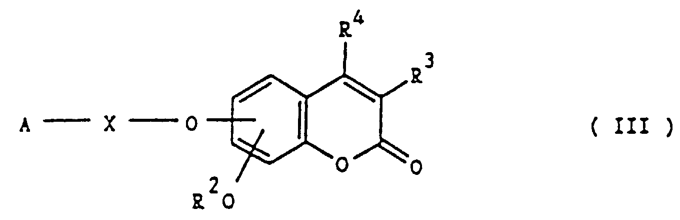 dans laquelle X, R², R³ et R⁴ ont les significations données ci-dessus et où A est un groupe partant, qui est choisi parmi l'ensemble comprenant les radicaux chloro, bromo, iodo, alkylsulfonyloxy, trifluorométhylsulfonyloxy, éventuellement phénylsulfonyloxy substitué par des substituants alkyle, nitro ou halogéno, avec un composé de formule générale V, 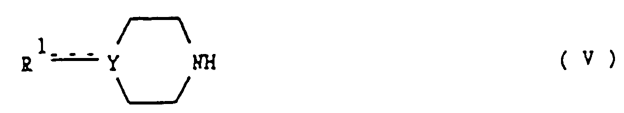 dans laquelle R¹ et Y ont les significations données ci-dessus, ou bien
b) on fait réagir un composé de formule générale VI, 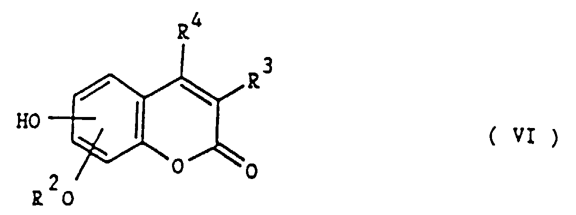 dans laquelle R², R³ et R⁴ ont les significations données ci-dessus, avec un composé de formule générale VII, formule générale VII, 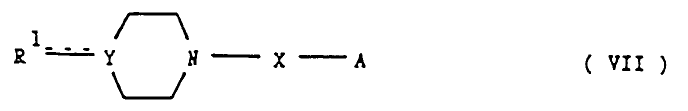 dans laquelle X, Y et R¹ ont les significations données ci-dessus, et A a les significations données ci-dessus en a)
ce après quoi les composés obtenus selon a) ou b) sont convertis éventuellement en leurs composés d'addition avec un acide, acceptables d'un point de vue physiologique.

2. Procédé selon la revendication 1, caractérisé en ce que la réaction s'effectue dans tous les cas en présence d'une base, qui est choisie parmi l'ensemble comprenant les carbonates alcalins, les carbonates alcalino-terreux, les hydrogénocarbonates, les hydrures, les alcoolates, les hydroxydes et les amines tertiaires.

3. Procédé pour préparer les composés de formule générale I, dans lesquels le groupe X est un groupe 2-hydroxypropyle, caractérisé en ce qu'on fait réagir un composé de formule générale IV, 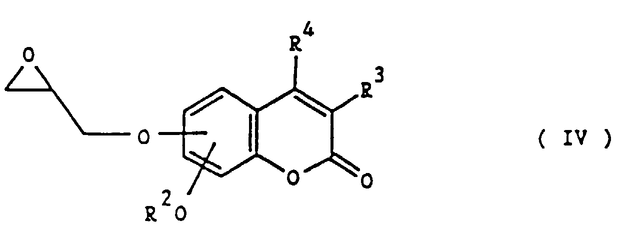 dans laquelle R², R³ et R⁴ ont les significations données dans la revendication 1,
avec un composé de formule générale V, 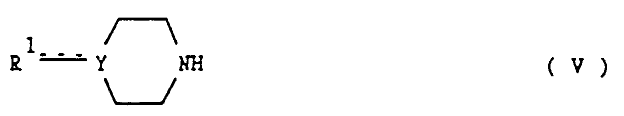 dans laquelle R¹ et Y ont les significations données dans la revendication 1.

4. Procédé selon l'une des revendications 1 à 3, caractérisé en ce que la réaction est mise en oeuvre en présence d'un solvant inerte vis-à-vis des réactants, choisi parmi l'ensemble comprenant les alcanols, les méthylbenzènes, les éthers aliphatiques et cycloaliphatiques, les dialkylamides d'acides carboxyliques, les tétraalkylurées, les cétones et les sulfoxydes.

5. Procédé selon l'une des revendications 1 à 4, caractérisé en ce que la réaction est mise en oeuvre en présence d'un catalyseur choisi parmi l'ensemble comprenant les iodures alcalins et alcalino-terreux.

6. Procédé selon l'une des revendications 1 à 5, caractérisé en ce que la réaction est mise en oeuvre à une température comprise entre la température ambiante et 130°C, de préférence à une température inférieure à 100°C, et sous une pression comprise entre la pression normale et 10⁷ Pa.

7. Procédé selon l'une des revendications 1 à 6, caractérisé en ce que la réaction est mise en oeuvre sous une atmosphère d'un gaz protecteur tel que N₂ ou Ar.

8. Procédé pour préparer des produits intermédiaires réactifs de formule générale XI 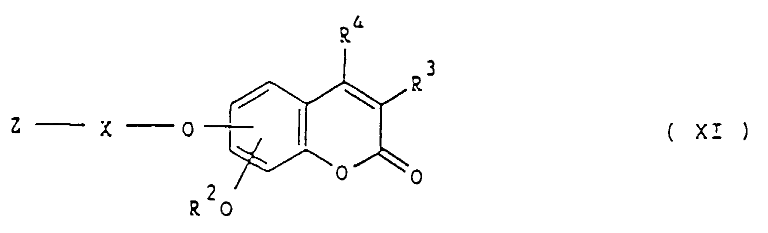 dans laquelle R², R³ et R⁴ ont les significations données dans la revendication 1, et
X est un groupe alkylène ayant de 2 à 5 atomes de carbone, et
Z est un groupe hydroxyle ou méthanesulfonyloxy, caractérisé en ce que un composé de formule générale VI 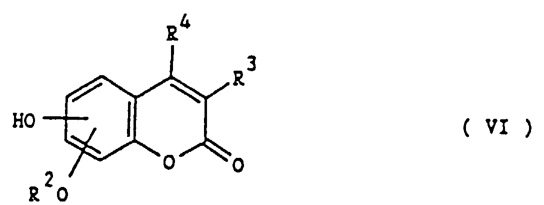
dans laquelle R², R³ et R⁴ ont les significations données dans la revendication 1, réagit avec un alcool halogéné correspondant (Hal-X-OH), et est ensuite éventuellement soumis à une réaction plus poussée avec le chlorure de méthanesulfonyle.

9. Procédé pour préparer des produits intermédiaires réactifs de formule générale (XI) 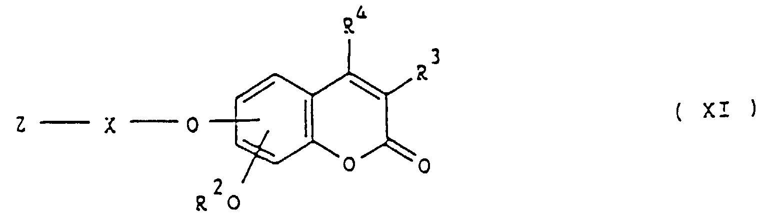 dans laquelle R², R³ et R⁴ ont les significations données dans la revendication 1, et
Z et X représentent ensemble un groupe oxirannylméthylène, à l'exception de la 7-méthoxy-6-(oxirannylméthoxy)-2H-1-benzopyranne-2-one et de la 7-méthoxy-4-méthyl-6-(oxirannylméthoxy)-2H-1-benzopyranne-2-one, caractérisé en ce qu'on fait réagir un composé de formule générale VI 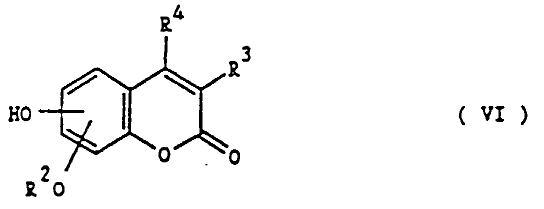 dans laquelle R², R³ et R⁴ ont les significations données dans la revendication 1, avec de l'épichlorhydrine ou de l'épibromhydrine.

10. Procédé pour préparer des produits intermédiaires réactifs de formule générale IX, 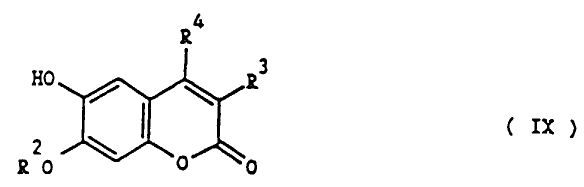 dans laquelle R², R³ et R⁴ ont les significations données dans la revendication 1 mais R⁴ n'est pas un hydrogène, caractérisé en ce qu'on fait réagir une 2-alcoxyhydroquinone de formule 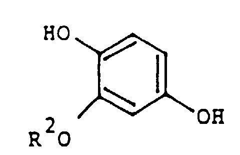 avec un ester d'un acide β-cétocarboxylique de formule générale X, 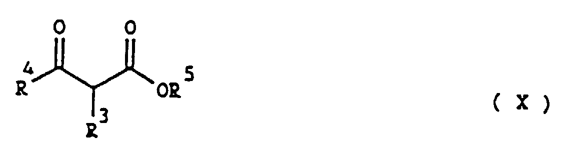 dans laquelle R³ et R⁴ ont les significations données ci-dessus, et R⁵ est un radical alkyle inférieur,
en présence d'un catalyseur acide.

11. Procédé selon la revendication 10, caractérisé en ce qu'on utilise un acide minéral ou un acide de Lewis comme catalyseur et simultanément comme solvant, la réaction étant mise en oeuvre à une température comprise entre 0 et 60°C.
